# EUROPÄISCHE PATENTSCHRIFT

(11) **EP 0 966 536 B1**
(45) Veröffentlichungstag und Bekanntmachung des Hinweises auf die Patenterteilung: **26.04.2006**
(21) Anmeldenummer: 98903943.3
(22) Anmeldetag: 27.02.1998
(51) Int. Cl.: C12N 15/57, C12N 9/64, A61K 38/48

(54) **FAKTOR X-ANALOGE MIT MODIFIZIERTER PROTEASESPALTSTELLE**
FACTOR X ANALOGUES WITH A MODIFIED PROTEASE CLEAVAGE SITE
ANALOGUES DU FACTEUR X DONT LE SITE DE CLIVAGE DONNANT UNE PROTEASE EST MODIFIE

(30) Priorität: 27.02.1997 AT 33597
(43) Veröffentlichungstag der Anmeldung: 29.12.1999
(73) Patentinhaber: Baxter Aktiengesellschaft, 1221 Wien (AT)
(72) Erfinder: HIMMELSPACH, Michele, CH- 2562 Port (CH); SCHLOKAT, Uwe, A-2304 Orth/Donau (AT); DORNER, Friedrich, A-1230 Wien (AT); FISCH, Andreas, CH-9000 St. Gallen (CH); EIBL, Johann, A-1180 Wien (AT)
(74) Vertreter: Alge, Daniel
(86) Internationale Anmeldenummer: PCT/AT1998/000045
(87) Internationale Veröffentlichungsnummer: WO 1998/038317

(56) Entgegenhaltungen:
- WOLF D L ET AL: "Design of constructs for the expression of biologically active recombinant human factors X and Xa. Kinetic analysis of the expressed proteins." JOURNAL OF BIOLOGICAL CHEMISTRY., Bd. 266, Nr. 21, 25.Juli 1991, MD US, Seiten 13726-13730, XP002065182 in der Anmeldung erwähnt
- LEYTUS S ET AL: "Gene for human factor X: a blood coagulation factor whose gene organizytion is essentially identical with that of factor IX and protein c" BIOCHEMISTRY, Bd. 25, Nr. 18, September 1986, Seiten 5098-5102, XP002065183 in der Anmeldung erwähnt

## Beschreibung

Die Erfindung betrifft Faktor X-Analoge mit einer Modifikation im Bereich des Aktivierungspeptids, eine Präparation enthaltend die erfindungsgemäßen Faktor X-Analoge sowie ein Verfahren zur Herstellung von einzelkettigen und zweikettigen Faktor X-Analogen.

Nach Initiierung des Blutgerinnungsprozesses verläuft die Gerinnungskaskade durch die sequentielle Aktivierung von verschiedenen Proenzymen (Zymogenen) im Blut in ihre aktive Formen, den Serinproteasen. Dazu gehören u.a. Faktor XII/XIIa, Faktor XI/XIa, Faktor IX/IXa, Faktor X/Xa, Faktor VII/VIIa und Prothrombin/Thrombin. Die meisten dieser Enzyme sind im physiologischen Zustand nur aktiv, wenn sie in einem Komplex an einer Membranoberfläche assoziiert sind. Ca-Ionen sind in viele dieser Prozesse involviert. Die Blutgerinnung folgt entweder dem intrinsischen Weg, bei dem alle Proteinkomponenten im Blut vorhanden sind, oder dem extrinsischen Weg, bei dem der Zellmembran-Gewebefaktor eine kritische Rolle spielt. Der Wundverschluß erfolgt schließlich durch die Spaltung von Fibrinogen zu Fibrin durch Thrombin.

Der Prothrombinase-Komplex ist verantwortlich für die Aktivierung von Prothrombin zu Thrombin. Thrombin ist ein wichtiges Enzym, das sowohl als Prokoagulant als auch als Antikoagulant wirken kann. Der Prothrombinase-Komplex, an dem u.a. Faktor Va (als Cofaktor) und Faktor Xa (als Serinprotease) beteiligt sind, assembliert in einer Ca-abhängigen Assoziation an der Oberfläche von Phospholipiden. Es wird diskutiert, daß dabei die katalytische Komponente des Prothrombinase-Komplexes Faktor Xa ist.

Faktor X (Stuart/Prower-Faktor) ist ein Vitamin K-abhängiges Koagulationsglykoprotein, das durch die intrinsische und extrinsische Blutgerinnungskaskade aktiviert werden kann. Das primäre Translationsprodukt von Faktor X (pre-pro-FX) besitzt 488 Aminosäuren und wird von der Leber oder humanen Hepatoma-Zellen zunächst als einzelkettiges 75 kD Vorläuferprotein synthetisiert. Im Plasma liegt Faktor X weitgehend als zweikettiges Molekül vor (Fair et al, 1984, Blood 64:194-204).

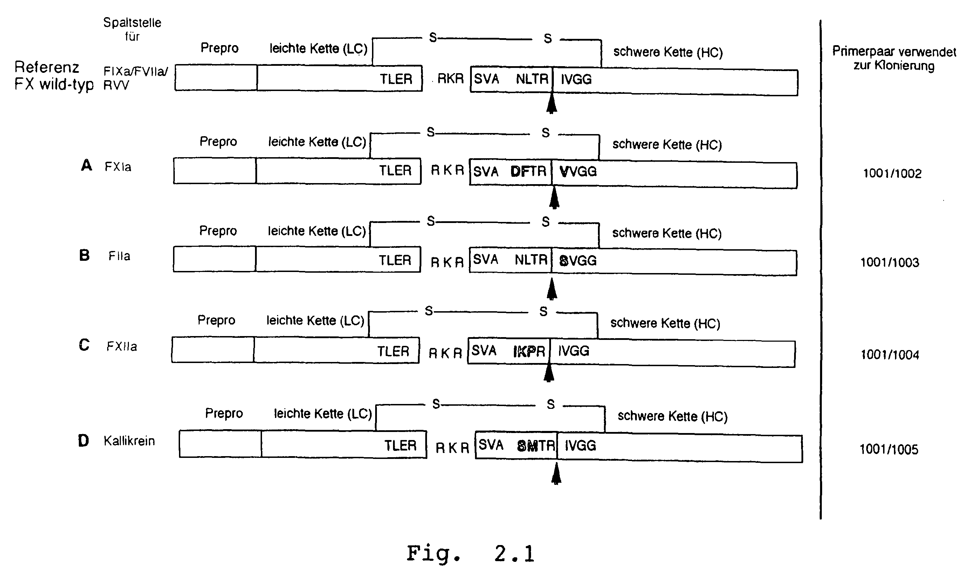

Während der Biosynthese wird nach Abspaltung der pre-Sequenz durch eine Signalpeptidase (zwischen Ser23/Leu24) und des Pro-peptides (zwischen Arg40/Ala41) das einzelkettige Faktor X-Molekül durch Prozessierung und Entfernung des Tripeptides Arg180-Lys181-Arg-182 in die zweikettige Form, bestehend aus der ca. 22 kD leichten Kette und der ca. 50 kD schweren Kette, die miteinander über eine Disulfid-Brücke verbunden sind, gespalten (Figur 2). Faktor X zirkuliert im Plasma daher als zweikettiges Molekül.

Während des Blutgerinnungsprozesses wird Faktor X vom inaktiven Zymogen zur aktiven Protease Faktor Xa durch limitierte Proteolyse konvertiert, wobei die Aktivierung von Faktor X zu Faktor Xa in einem von 2 membrangebundenen Komplexen erfolgen kann: dem extrinsischen Faktor VIIa/Gewebefaktor-Komplex oder dem intrinsischen Faktor VIIIa-Faktor IXa-Phospholipid-Ca-Komplex oder "Tenase-Komplex" (Mertens et al-, 1980, Biochem. J. 185:647-658). Eine proteolytische Spaltung zwischen Aminosäuren Arg 234/Ile 235 führt zur Freisetzung eines 52 Aminosäuren langen Aktivierungspeptids vom N-Terminus der schweren Kette und damit zur Bildung des aktiven Enzyms , Faktor Xa . Das katalytische Zentrum des Faktor Xa ist auf der schweren Kette lokalisiert.

Die Aktivierung über den Faktor VIIa-TF(extrinsischen)-Komplex führt zur Bildung von Faktor Xaα (35 kD) und Faktor Xaβ (31 kD), wobei bei geringen Konzentrationen von Faktor Vlla im Komplex auch ein Polypeptid von 42 (kD) auftritt. Die Bildung von Faktor Xaα erfolgt über eine Spaltung bei Arg234/Ile 235 der schweren Kette und repräsentiert die Aktivierung von Faktor X zu Faktor Xa. Das Auftreten von Faktor Xaβ resultiert vermutlich aus einer autokatalytischen Spaltung bei Arg469/Gly470 im C-Terminus der schweren Kette von Faktor Xaα und der Abspaltung eines 4 . 5 kD-Peptides . Faktor Xaβ besitzt ebenfalls wie Faktor Xaα katalytische Aktivität. Es wurde jedoch gezeigt, dass durch die Spaltung von Faktor Xaα zu Faktor Xaβ eine Plasminogen-Bindungsstelle entsteht und Faktor Xaβ gegebenenfalls fibrinolytische Aktivität aufweist bzw. an Fibrinolyse als Cofaktor beteiligt ist. Die Umwandlung von Faktor Xa*α* zu Faktor Xa*β* ist jedoch langsamer als die Bildung von Thrombin, wodurch eine Initiierung der Fibrinolyse vor Ausbildung eines Blutklots verhindert wird (Pryzdial et al., 1996, J. Biol. Chem. 271:16614-16620; Pryzdial et al., 1996, J. Biol. Chem. 271:16621-16626).

Das 42 kD-Polypeptid resultiert aus einer Prozessierung im C-Terminus der schweren Kette zwischen Arg469/Gly470 ohne vorherige Prozessierung zwischen Arg234/Ile 235. Dieses Intermediat besitzt ebenso wie ein Faktor Xaγ-Fragment, das durch Proteolyse bei Lys370 entsteht, keine katalytische Aktivität (Mertens et al., 1980, Biochem. J. 185:647-658; Pryzdial et al., 1996, J. Biol. Chem. 271:16614-16620).

Die Aktivierung von Faktor X im intrinsischen Weg wird katalysiert durch den Faktor IXa-Faktor VIIIa-Komplex. Während der Aktivierung werden die gleichen Prozessierungsprodukte erhalten, jedoch wird das Faktor Xa*β*-Produkt in einem höheren Ausmaß erhalten als andere Faktor X-Prozessierungsprodukte (Jesty et al., 1974, J. Biol. Chem. 249:5614).

In vitro kann Faktor X beispielsweise durch Russell's Viper Venom (RVV) oder Trypsin (Bajaj et al., 1973, J.Biol. Chem. 248:7729-7741) oder gereinigte physiologische Aktivatoren, wie FVIIa/TF-Komplex oder Faktor IXa/Faktor VIIIa-Komplex aktiviert werden (Mertens et al., 1980, Biochem. J. 185:647-658).

Kommerziell erhältliche Faktor X-Produkte aus Plasma enthalten zumeist eine Mischung aus Faktor Xa*α* und Faktor Xa*β*, da nach Aktivierung von Faktor X zu Faktor Xa in erster Linie Faktor Xaα entsteht, der in einem autokatalytischen Prozeß wiederum zu Faktor Xaβ gespalten wird. Um ein einheitliches Faktor Xa-Produkt mit hoher molekularer Integrität herzustellen, wurde in der EP 0 651 054 vorgeschlagen, Faktor X mit RW über einen längeren Zeitraum zu aktivieren, sodaß das resultierende Endprodukt im wesentlichen Faktor Xaβ enthielt. Sowohl die Nebenprodukte, beispielsweise Faktor Xaα, als auch die Protease wurden anschließend durch mehrere chromatographische Schritte entfernt.

Die cDNA für Faktor X wurde isoliert und charakterisiert (Leytus et al., 1984, Proc. Natl. Acad. Sci., USA, 82:3699-3702; Fung et al., 1985, Proc. Natl. Acad. Sci., USA, 82:3591-3595). Humaner Faktor X wurde in vitro in verschiedenen Zelltypen, wie humanen embryonalen Nierenzellen oder CHO-Zellen exprimiert (Rudolph et al., 1997, Prot.Expr.Purif.10: 373-378; Wolf et al., 1991, J. Biol.Chem. 266:13726-13730). Es wurde jedoch festgestellt, daß bei der rekombinanten Expression von humanem Faktor X die Prozessierung an Position Arg40/Ala41 im Gegensatz zur in vivo-Situation ineffizient erfolgt und unterschiedliche N-Termini an der leichten Kette von Faktor X entstehen (Wolf et al., 1991, J. Biol.Chem. 266:13726-13730). Rekombinanter Faktor X (rFX) wurde durch RVV in vitro zu rFaktor Xa (rFXa) aktiviert oder rFXa direkt exprimiert, wobei das Aktivierungspeptid von Aminosäure 183 bis Aminosäure 234 deletiert und durch ein Tripeptid ersetzt wurde, um eine Prozessierung unmittelbar in eine zweikettige rFXa-Form zu ermöglichen. Gereinigter rFX wurde zu etwa 70% in leichte und schwere Kette prozessiert, während die verbleibenden 30% einzelkettigen rFX mit 75 kD darstellten. Direkte Expression von rFXa führte zwar zur Bildung von aktivem Faktor Xa, jedoch auch zu inaktiven Intermediaten. Wolf et al. (1991, J. Biol. Chem. 266:13726-13730) stellten weiterhin eine verringerte Aktivität von rekombinantem Faktor X fest, die sie auf die schlechtere Aktivierbarkeit des rFX durch RVV sowie auf die inaktive Population an Proteinen und Polypeptiden des einzelkettigen Vorläufermoleküls zurückführten. Insbesondere fanden sie eine hohe Instabilität von rFXa bei Expression durch rekombinante Zellen, was sie auf die hohe Autoproteolyserate zurückführten.

Um die Funktion des C-terminalen Peptides von Faktor Xaα zu untersuchen, führten Eby et al. (1992, Blood 80 (Suppl. 1): 1214 A) ein Stopcodon an Position Gly430 der Faktor X-Sequenz ein. Sie fanden jedoch keinen Unterschied zwischen der Aktivierungsrate von Faktor Xa (FXa*α*) mit b-Peptid oder einer Deletionsmutante ohne β-Peptid (FXa*β*).

Faktor Xa ist ein wichtiger Bestandteil des Prothrombinase-Komplexes und könnte daher zur Behandlung von Patienten mit Störungen der Blutgerinnung, beispielsweise bei Hämophilie, verwendet werden.

Insbesondere die Behandlung von Hämophilie-Patienten mit Faktor VIII- oder Faktor IX-Defizienz mit Faktoren-Konzentraten, hergestellt aus Plasma, wird bei längeren Therapiezeiten oft dadurch kompliziert, daß inhibitorische Antikörper gegen diese Faktoren gebildet werden. Es wurde daher eine Reihe von Alternativen entwickelt, um Hämophilie-Patienten mit Faktoren mit einer By-pass-Aktivität zu behandeln. So wurde die Verwendung von ProthrombinKomplex-Konzentrat, partiell aktiviertem Prothrombinase-Komplex (APPC), Faktor VIIa oder FEIBA vorgeschlagen. Kommerzielle Präparate mit Faktor VIII-Bypass-Aktivität (FEIBA) sind beispielsweise FEIBA® oder Autoplex®. FEIBA etwa enthält vergleichbare Einheiten an Faktor II, Faktor VII, Faktor IX, Faktor X und FEIBA, geringe Mengen an Faktor VIII und Faktor V, sowie Spuren von aktivierten Koagulationsfaktoren, wie Thrombin und Faktor Xa bzw. einen Faktor mit Faktor X-ähnlicher Aktivität (Elsinger, 1982, Activated Prothrombin Complex Concentrates. Ed. Mariani, Russo, Mandelli, p.77-87). Elsinger weist insbesondere auf die Bedeutung einer "Faktor Xa-like"-Aktivität in FEIBA hin. Faktor VIII-Bypass-Aktivität wurde von Giles et al. (1988, British J. Haematology 9:491-497) für eine Kombination von gereinigtem Faktor Xa und Phospholipiden im Tiermodell gezeigt.

Es besteht daher ein großer Bedarf und eine Reihe von verschiedenen Anwendungsgebieten für Faktor X/Xa oder Faktor X/Xa-ähnlichen Proteinen, entweder allein oder als Bestandteil eines Koagulationskomplexes in der Blutstillungstherapie.

Die Halbwertszeit von Faktor Xa ist gegenüber dem Zymogen sowohl in vivo als auch in vitro stark herabgesetzt. So kann etwa Faktor X in Glycerin 18 Monate stabil aufbewahrt werden, während Faktor Xa unter gleichen Bedingungen nur 5 Monate stabil ist (Bajaj et al., 1973, J. Biol. Chem. 248:7729-2241) bzw. in Glycerin bei 4°C nach 8 Monaten eine Reduktion der Aktivität um mehr als 60 % zeigt (Teng et al., 1981, Thrombosis Res. 22: 213-220). Die Halbwertszeit von Faktor Xa beträgt lediglich 30 Sekunden im Serum.

Aufgrund der Instabilität von Faktor Xa wurde vorgeschlagen, Faktor X-Präparate zu verabreichen (US 4,501,731). Bei lebensbedrohenden Blutungen, insbesondere bei Hämophilie-Patienten, ist jedoch ein Verabreichung von Faktor X wirkungslos, da durch das Fehlen des funktionellen "Tenase-Komplexes" im intrinsischen Blutgerinnungsweg keine ausreichende Aktivierung von Faktor X zu Faktor Xa erfolgen kann und die Aktivierung über den extrinsischen Weg oftmals zu langsam erfolgt, um eine rasche Wirkung zu erzielen. Zudem ist bei Hämophilie-Patienten ausreichend Faktor X vorhanden, der jedoch im Vergleich zu Faktor Xa eine 1000fach geringere Prothrombinase-Aktivität besitzt. In solchen Fällen ist es erforderlich, aktivierten Faktor Xa direkt, gegebenenfalls zusammen mit Phospholipiden, wie bei Giles et al. (1988. British J. Haematology 9:491-497) beschrieben oder mit anderen Koagulationsfaktoren etwa mit Faktor VIII By-pass-Aktivität, zu verabreichen.

Bei der Herstellung von Faktor Xa aus Faktor X erfolgte die Aktivierung bisher zumeist über unphysiologische Aktivatoren tierischen Ursprungs, wie RVV oder Trypsin, wobei jedoch absolut sicher gestellt sein mußte, daß das Endprodukt vollständig frei von diesen Proteasen ist. Wie oben schon erwähnt, werden bei der Aktivierung von Faktor X zu Faktor Xa eine Vielzahl teilweise auch inaktiver Intermediate gebildet (Bajaj et al., 1973, J.Bio. Chem. 248:7729-7741, Mertens et al., 1980, Biochem. J. 185: 647-658). Die Anwesenheit solcher Intermediate führt zu einer Erniedrigung der spezifischen Aktivität des Produktes und gegebenenfalls auch zu solchen Intermediaten, die als Antagonisten der aktiven Serinprotease fungieren können. Zur Herstellung eines einheitlichen, reinen Produktes mit hoher spezifischer Aktivität sind daher bei konventionellen Methoden aufwendige Verfahren zur Aktivierung sowie zur chromatographischen Reinigung erforderlich.

Aufgabe der vorliegenden Erfindung ist es daher eine Präparation zur Verfügung zu stellen, die ein Polypeptid mit Faktor X/Xa-Aktivität enthält, das eine hohe Stabilität aufweist und das ohne die Verwendung einer der üblichen Proteasen, insbesondere tierischen Ursprungs, wie beispielsweise RVV oder Trypsin zu Faktor Xa aktiviert werden kann. Ein weiteres Ziel ist es eine pharmazeutische Präparation mit Faktor VIII-Bypass-Aktivität bereitzustellen.

Die Aufgabe wird erfindungsgemäß dadurch gelöst, daß ein Faktor X-Analogon zur Verfügung gestellt wird, das eine Modifikation im Bereich der natürlicherweise vorkommenden Faktor Xa-Aktivierungsspaltstelle aufweist. Die Modifikation im Bereich der Aktivierungsspaltstelle ist eine neue, nicht-natürlicherweise an dieser Position im Polypeptid vorkommende Erkennungs- bzw. Prozessierungsstelle für eine Protease, die das Polypeptid normalerweise nicht an dieser Stelle spaltet.

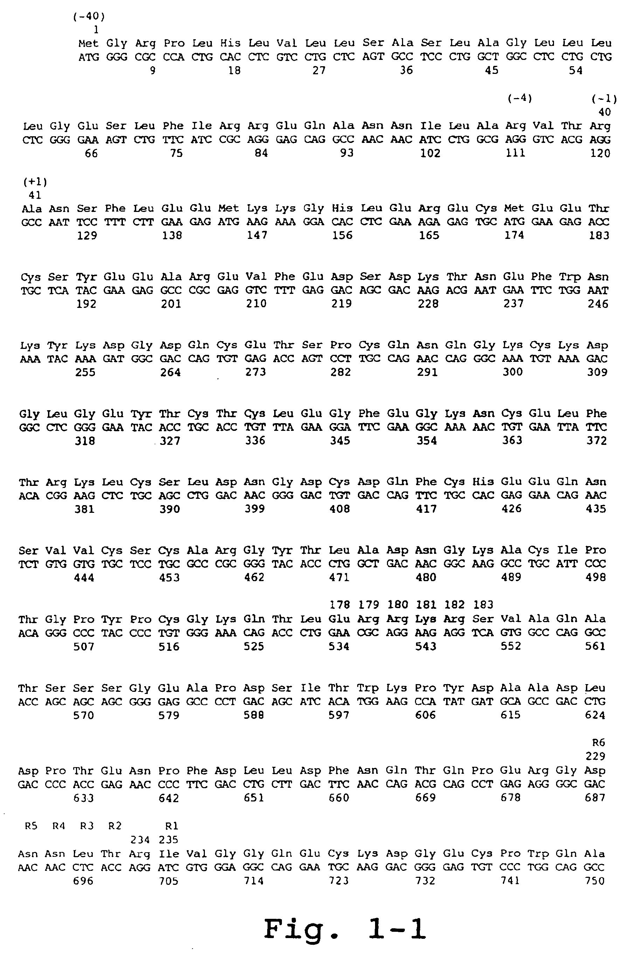

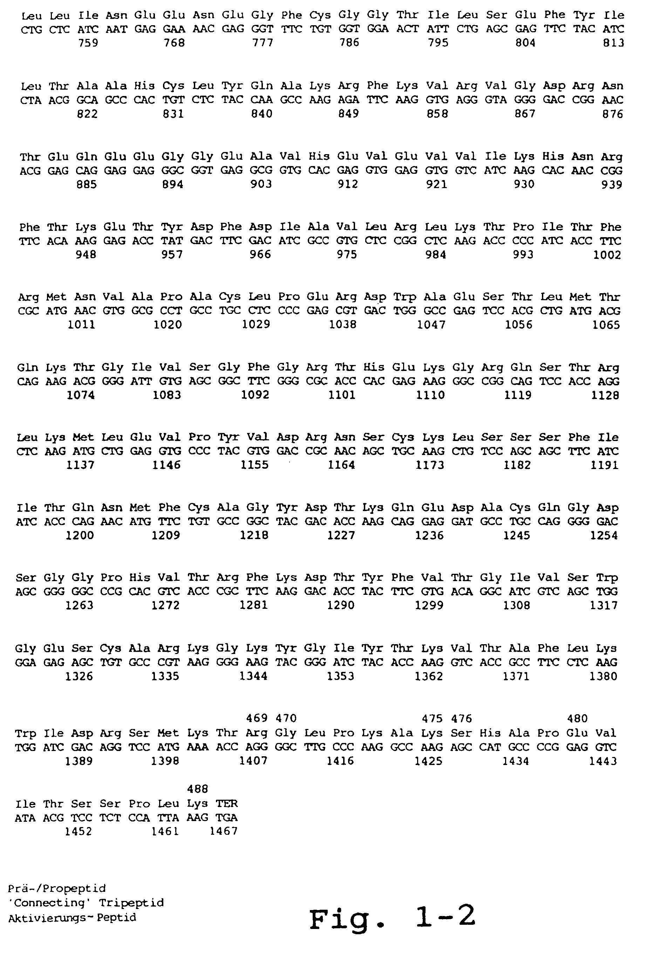

Das erfindungsgemäße Faktor X-Analogon weist dabei insbesondere im Aktivierungspeptid, das bei der Aktivierung von Faktor X zu Faktor Xa abgespalten wird, eine Modifikation auf. Die Modifikation betrifft dabei mindestens eine Aminosäure innerhalb der Aminosäuresequenz des Aktivierungspeptids des Faktor X. Die Modifikation ist dabei besonders im C-terminalen Bereich des Aktivierungspeptids und stellt mindestens einen Austausch mindestens einer Aminosäure zwischen Position Gly228 und Arg234 der Faktor X-Aminosäuresequenz dar. Die Position der Aminosäuren ist dabei bezogen auf die Numerierung gemäß der in Fig. 1 dargestellten Sequenz beginnend mit Met1 und endend mit Lys488.

Die Modifikation im erfindungsgemäßen Faktor X-Analogon ist vorzugsweise ein Austausch einer an dieser Stelle vorkommenden Faktor VIIa- bzw. Faktor IXa-Prozessierungsstelle gegen eine alternative Spaltstelle einer anderen Protease. Die Modifikation kann dabei eine Substitution mindestens einer Aminosäure oder eine Insertion einer Peptidsequenz, die eine Proteaseerkennungs- bzw. Spaltstelle darstellt, sein. Die Modifikation im erfindungsgemäßen Faktor X-Analogon ist dabei vorzugsweise derart, daß sie für eine Protease aus der Gruppe der Endoproteasen, wie etwa Kexin/Kex2, Furin/PACE, PC1/PC3, PC2, PC4, PACE 4, LPC/PC7 (wie in Barr et al., 1991, Cell 66:1-3 oder der US 5,460,950 beschrieben), der Serinproteasen, wie etwa Faktor XIIa, Faktor XIa, Faktor IIa, Faktor Xa oder von Kallikrein, oder einem Derivat dieser Proteasen, eine Erkennungs- bzw. Spaltsequenz darstellt.

Die Modifikation ist vorzugsweise so ausgewählt, daß die Prozessierung durch eine dieser Proteasen zu einem dem nativen Faktor Xa entsprechenden Polypeptid führt, das im wesentlichen der natürlich vorkommenden Faktor Xa-Sequenz gleicht und ebenfalls Faktor Xa-Aktivität aufweist.

Um eine optimale Prozessierung zu erreichen, kann es in einzelnen Fällen notwendig sein, zusätzlich die Aminosäure Ile235 auszutauschen. Vorzugsweise sollte die NH2-terminale Aminosäure Isoleucin der schweren Kette jedoch nach Aktivierung erhalten bleiben, da dieser Aminosäure bei der Bildung der Substratbindungstasche eine wesentliche Funktion zukommt (Watzke et al., 1995, Molecular Basis of Thrombosis and Hemostasis ed. Katherine High & Harold Roberts). Die erfindungsgemäßen Faktor X-Analoge zeigen einen strukturellen Unterschied, insbesondere auf Aminosäure-Ebene im Vergleich zur nativen Faktor X-Sequenz, besitzen jedoch eine vergleichbare Aktivierbarkeit, wie natürlich vorkommender Faktor X bzw. nach Aktivierung Faktor Xa-Aktivität.

Die Erfindung stellt dabei beispielhaft eine Anzahl von Faktor X-Analogen zur Verfügung, die eine Modifikation im Aktivierungspeptid in bezug auf die natürlich vorkommende Faktor X-Sequenz und eine veränderte Proteasespezifität aufweisen.

Modifikationen können an einer oder mehreren Positionen im Bereich zwischen Aminosäure Gly228 und Arg234, und gegebenenfalls Ile235, bezogen auf die Faktor X-Sequenz mit der Numerierung mit Met1 bis Lys 488 gemäß Fig. 1, sein. Aminosäuresubstitutionen können dabei sein an Position Ile235 (R1), Arg234, Thr233 (R2), Leu232 (R3), Asn231 (R4), Asn230 (R5) und Asp229 (R6), wobei jedoch vorzugsweise Arg234 unverändert bleibt.

Die erfindungsgemäßen Faktor X-Analogon enthalten vorzugsweise eine Faktor X-Sequenz mit Gly 228-R6-R5-R4-R3-R2-Arg234 mit R1= Ile, Val, Ala, Ser oder Thr; R2= Thr, Pro, Gly, Lys oder Arg; R3= Leu, Phe, Lys, Glu, Met, Gln, Ser, Val, Arg oder Pro; R4= Asn, Asp, Ile, Ser, Met, Pro, Thr, Lys oder Arg; R5= Asn, Lys, Ser, Glu, Ala, Gln, His oder Arg und R6= Asp, Phe, Thr, Arg, Leu oder Ser, darstellt.

Bevorzugte Ausführungsformen der erfindungsgemäßen Faktor X-Analoge sind dabei Faktor X-Analoge, die eine Modifikation aufweisen mit
a) R1=Val, R2=Thr, R3=Phe, R4=Asp, R5=Asn und gegebenenfalls R6=Phe (Figur 2 A) und durch Faktor XIa prozessiert wird;
b) R1=Ser, R2=Arg, R3=Thr, R4=Leu (Fig. 2 B) und durch FIIa prozessiert wird;
c) R1=Ile, R2=Pro, R3=Lys, R4=Ile und gegebenenfalls R5=Lys und/oder R6=Thr (Fig. 2 C) oder 1R=Ile, R2=Thr, R3=Ser, R4=Thr und gegebenenfalls R5=Lys und/oder R6=Thr (Fig. 2 I) und durch Faktor XIIa prozessiert werden;
d) R1=Ile, R2=Thr, R3=Met, R4=Ser und gegebenenfalls R5=Ser und/oder R6=Leu (Fig. 2 D) und durch Kallikrein prozessiert wird;
e) R1=Ile, R2=Gly, R3=Gln, R4=Pro und gegebenenfalls R5=Lys und/oder R6=Ser (Fig. 2 H) oder R1=Ile, R2=Thr, R3=Lys und R4=Met (Fig. 2 E) oder R1=Ile, R2=Gly, R3=Glu und R4=Ile (Fig. 2 F) und durch Faktor Xa prozessiert werden;
f) R1=Ile, R2=Lys, R3=Arg, R4=Arg und gegebenenfalls R5=Glu und/oder R6=Leu oder
   R1=Ile, R2=Thr, R3=Val, R4=Arg und gegebenenfalls R5=Ala und/oder R6=Leu oder
   R1=Ile, R2=Arg, R3=Val, R4=Arg und gegebenenfalls R5=Gln und/oder R6=Leu oder
   R1=Ile, R2=Arg, R3=Arg, R4=Arg und gegebenenfalls R5=His und/oder R6=Leu oder
   R1=Ile, R2=Lys, R3=Pro, R4=Arg und gegebenenfalls R5=Asn und/oder R6=Leu oder
   Rl=Ile, R2=Lys, R3=Arg, R4=Ile und gegebenenfalls R5=Arg und/oder R6=Leu oder
   R1=Ile, R2=Lys, R3=Ser und R4=Arg oder
   R1=Ile, R2=Thr, R3=Val und R4=Arg oder
   R1=Ile, R2=Lys, R3=Leu und R4=Arg (siehe alle Fig. 2 G),
wobei die unter f) genannten durch eine bibasische Endoprotease, wie Furin, PACE, Kexin/Kex2, Furin/PACE, PC1/PC3, PC2, PC4, PACE 4, LPC/PC7 oder einem Derivat einer dieser Proteasen prozessiert werden.

Eine mögliche Auswahl von Modifikationen und Aminosäureaustauschen, die zu einer veränderten Proteasespezifität führen, sind in Figur 2 A-I angeführt.

Die Modifikationen können dabei beispielsweise durch gerichtete in vitro Mutagenese oder PCR oder andere aus dem Stand der Technik bekannte gentechnische Methoden durchgeführt werden, die dazu geeignet sind, spezifisch eine DNA-Sequenz zu verändern, um gezielt Aminosäurenaustausche auszuführen.

Die Aktivierung des erfindungsgemäßen Faktor X-Analogons zu einem nativen Faktor Xa bzw. einem Faktor Xa-Analogon erfolgt gemäß der vorliegenden Erfindung vorzugsweise durch eine Protease, ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor XIIa, Faktor XIa, Faktor Xa, Faktor IIa oder von Kallikrein, oder einem Derivat dieser Proteasen.

Eine der Schwierigkeiten bei der Herstellung von aktivem Faktor Xa ist seine Instabilität, da durch Autokatalyse neben Faktor Xaα und Faktor Xaβ auch andere, inaktive Intermediate entstehen. Zur Herstellung von im wesentlichen intakten, aktiven Faktor X/Xa- bzw. Faktor X/Xa-ähnlichen Molekülen wäre es daher wünschenswert, nur solche Proteine zu erhalten, die zu stabilen Endprodukten führen.

Es ist bekannt, daß eine bevorzugte Spaltstelle für die Prozessierung von Faktor Xa*α* (Fxa*α*) zu Faktor Xa*β* (Fxa*β*) zwischen Arg469/Gly470 liegt. Aufgrund von Untersuchungen von Eby et al. (1992, Blood. Vol. 80, Suppl. 1, 1214) wird neben einem prominenten carboxyterminalen Peptid (Aminosäurereste 476-487) von Faktor X ein weiteres kürzeres Peptid (Aminosäurereste 474 bis 477) gefunden, das durch Autokatalyse von Faktor Xaα entsteht.

Um eine gezielte Prozessierung von intaktem Faktor X zu im wesentlichen aktivem Faktor Xa zu focussieren, ohne dabei inaktive Prozessierungs-Intermediate zu erhalten, weisen die erfindungsgemäßen Faktor X-Analoge gegebenenfalls weitere Modifikationen auf.

Das erfindungsgemäße Faktor X-Analogon weist daher gemäß einer besonderen Ausführungsform eine weitere Modifikation im C-terminalen Bereich der Faktor X-Aminosäuresequenz auf.

Gemäß einer Ausführungsform weist ein Faktor X-Analogon der oben beschriebenen Art ein intaktes *β*-Peptid (Fx*α*) auf. Das erfindungsgemäße Faktor X-Analogon besitzt dabei insbesondere eine Modifikation im Bereich der C-terminalen *β*-Peptidspaltstelle, die verhindert, daß nach Aktivierung von Faktor X zu Faktor Xa eine Abspaltung des *β*-Peptids von Faktor X erfolgt. Dadurch wird ein Faktor Xa-Molekül erhalten, das bis zu 100% als intaktes Faktor Xaα-Molekül isoliert werden kann.

Die Modifikation kann eine Mutation, Deletion oder Insertion im Bereich der Faktor X-Aminosäuresequenz zwischen Aminosäureposition Arg469 und Ser476 und gegebenenfalls von Lys 370 sein. Bevorzugt ist jedoch eine Aminosäuresubstitution, bei der durch den Aminosäureaustausch eine die Struktur und damit gegebenenfalls die Funktion und Aktivität des Proteins beeinflussende Faltung des Polypeptides nicht erfolgen kann.

Gemäß einer Ausführungsform weisen die erfindungsgemäßen Faktor X-Analoge einen Austausch einer der Aminosäuren an Position Arg469 und/oder Gly470, wobei Arg469 vorzugsweise gegen Lys, His oder Ile und Gly470 vorzugsweise gegen Ser, Ala, Val oder Thr ausgetauscht ist.

Die erfindungsgemäßen Faktor X-Analoge können neben einer Mutation an Position Arg469 und/oder Gly470 eine weitere Mutation an Position Lys370 und/oder Lys475 und/oder Ser476 aufweisen.

Durch eine Aminosäuresubstitution an einer dieser Positionen wird eine Prozessierung von Faktor Xa*α* zu Faktor Xa*β* bzw. Faktor Xaγ vermieden, da die natürlicherweise vorkommende(n) Prozessierungssequenz(en) derart modifiziert ist (sind), daß eine gegebenenfalls autokatalytische Abspaltung des Carboxy-terminalen Peptids nicht mehr erfolgen kann.

Gemäß einer anderen Ausführungsform weist das erfindungsgemäße Faktor X-Analogon eine Deletion des carboxyterminalen *β*-Peptids (FX*β*) auf. Ein solches Faktor X-Analogon kann hergestellt werden, indem eine cDNA kodierend für ein Faktor X-Analogon in einem rekombinanten Expressionssystem exprimiert wird, wobei nur die Sequenzen kloniert werden, die für die Aminosäuren Met1 bis Arg469 kodieren.

Gemäß einer weiteren Ausführungsform weist das erfindungsgemäße Faktor X-Analogon ein Translationsstopsignal im C-terminalen Bereich der Faktor X-Sequenz auf. Das Translationsstopsignal ist dabei vorzugsweise an einer Position, die einer nach natürlicher Prozessierung entstehenden C-terminalen Aminosäure folgt. Das Translationsstopsignal ist daher vorzugsweise an Position der Aminosäure 470 der Faktor X-Sequenz, damit das endständige Arg469 des Faktor Xa*β* erhalten bleibt. Dazu wird das für die Aminosäure Gly470 kodierende Kodon GGC gegen TAA, TAG oder TGA substituiert.

Ein weiterer Aspekt der vorliegenden Erfindung betrifft Faktor X-Analoge, die durch Behandlung mit einer entsprechenden Protease in vitro zu nativem Faktor Xa bzw. einem Faktor Xa-Analogon aktiviert werden. Abhängig vom Faktor X-Analogon, das eingesetzt und aktiviert wird, wird ein dem nativen Faktor Xa entprechendes und im wesentlichen identes Polypeptid erhalten bzw. ein Polypeptid, das zwar Faktor Xa-Aktivität aufweist, jedoch in bezug auf die native Faktor Xa-Sequenz Modifikationen aufweist, die jedoch nicht die biologische Aktivität beeinträchtigen. Bei Aktivierung von Faktor X-Analogen, die die Modifikation im Bereich des Aktivierungspeptids in der Sequenz des Aktivierungspeptids aufweisen, werden ausschließlich dem nativen Faktor Xa-Molekül entsprechende Polypeptide erhalten. Weist ein solches Faktor X-Analogon gegebenenfalls zusätzlich ein Translationsstopsignal im C-terminalen Bereich des β-Peptids auf, so werden Faktor Xaβ-homologe Moleküle gewonnen. Wird jedoch ein Faktor X-Analogon eingesetzt, das Modifikation(en) innerhalb der ß-Peptidsequenz aufweist, die dazu führt(en), daß das β-Peptid nicht abgespalten wird, so wird ein Faktor Xaα-Analogon mit einem Aminosäureaustausch im C-Terminus des Moleküls erhalten.

Die erfindungsgemäßen Faktor X-Analoge weisen ausschließlich Modifikationen auf, die die Spezifität für die Aktivierbarkeit ändern und nicht die Aktivität beeinflussen. Es werden daher in jedem Fall biologisch und funktionell aktive Faktor Xa-Moleküle bzw. Faktor Xa-Analoge gewonnen.

Die Aktivierung in vitro kann durch eine Protease ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor XIIa, Faktor XIa, Faktor Xa, oder von Kallikrein, oder einem Derivat dieser Proteasen erfolgen. Es liegt jedoch im Rahmen der vorliegenden Erfindung, jede beliebige Protease, außer RVV, Trypsin, Faktor IXa oder Faktor VIIa einzusetzen, sofern sie geeignet ist, das erfindungsgemäße Faktor X-Analogon zu Faktor Xa zu prozessieren.

Gemäß einer weiteren Ausführungsform der Erfindung enthält das Faktor X-Analogon eine Modifikation, die eine Aktivierung des Faktor X-Analogon zu Faktor Xa, vorzugsweise nativem Faktor Xa, in vivo erlaubt. "Nativer" Faktor Xa bedeutet in diesem Zusammenhang, daß der aktivierte Faktor Xa, abgeleitet vom erfindungsgemäßen Faktor X-Analogon, die dem nativem Faktor Xa entsprechende und homologe Aminosäuresequenz aufweist und Faktor Xa-Aktivität besitzt. Die Modifikation ist dabei so gewählt, daß eine Prozessierung von Faktor X zu Faktor Xa durch eine in vivo, also im Körper vorkommende Protease, vorzugsweise eine in der Blutgerinnungskaskade vorkommende Protease erfolgt. Die Protease kann dabei eine Protease ausgewählt aus der Gruppe der Serinproteasen, wie etwa Faktor IIa, Faktor XIIa, Faktor XIa, Faktor Xa oder Kallikrein sein. Faktor X-Analoge, die neben der Modifikation im Aktivierungspeptid eine Modifikation im C-terminalen Bereich des Faktor X-Moleküls aufweisen, werden, wie oben beschrieben, auch in vivo in die entsprechenden Faktor Xa-Analoge aktiviert.

Obwohl beispielsweise von Wolf et al. (1991, J. Biol. Chem. 266: 13726-137309) vermutet wurde, daß eine Endopeptidase wie Kex2, Furin oder PACE an der Prozessierung der von dieser Gruppe beschriebenen Faktor Xa-Deletionsmutante beteiligt ist, geben sie keinerlei Hinweise über den Einfluß einer dieser Proteasen bei der Prozessierung von Faktor X. Ebenso wird in der US 5,660,950 die rekombinante Herstellung von PACE und die Verwendung der Protease zur Verbesserung der Prozessierung von Vitamin-K abhängigen Proteinen beschrieben. In einer Reihe von Aufzählungen mit anderen Blutfaktoren wird auch Faktor X genannt, wobei jedoch diese Aussage verifizierende Daten fehlen.

Im Rahmen der vorliegenden Erfindung wurde zum erstenmal eindeutig gezeigt, daß eine der für den Reifungsprozeß von Faktor X notwendige Protease eine bibasische Endoprotease, insbesondere endogen vorkommendes Furin, ist. In vivo vermittelt die Endoprotease in erster Linie die Spaltung des einzelkettigen Faktor X-Moleküls in die reife Form bestehend aus schwerer und leichter Kette. In vitro vermittelt sie zusätzlich die Abspaltung der Faktor X Propeptid-Sequenz (Beispiel 2).

Erfindungsgemäße Faktor X-Analoge, die eine Proteasespaltstelle für eine nicht-natürlicherweise in einer Zelle vorkommende Protease aufweisen, werden durch selektive Prozessierungsreaktion nur an den Stellen gespalten, die auch im nativen Faktor X gespalten werden. Dadurch wird rekombinantes Faktor X-Molekül erhalten, das lediglich aus der 22 kD leichten Kette und der ca. 50 kD schweren Kette besteht und nicht durch unspezifische Prozessierung auftretende inaktive Faktor X-Moleküle aufweist. Diese modifizierten Faktor X-Moleküle werden, ebenso wie die nativen Faktor X-Moleküle, nicht durch die intrazelluläre Protease zu Faktor Xa aktiviert. Sie werden erst anschließend durch die entsprechenden Proteasen (vorzugsweise Serinproteasen oder Subtilisin-verwandte Proteasen) zu Faktor Xa aktiviert.

Gemäß einer Ausführungsform wird daher ein zweikettiges Faktor X-Analogon zur Verfügung gestellt.

Gemäß einer besonderen Ausführungsform werden Faktor X-Analoge bereitgestellt, die vorzugsweise in gereinigter Form als einzelkettige Moleküle vorliegen. Durch Expression von Faktor X-Analogon in einer für eine bibasische Protease defizienten Zellen, wird pro-Faktor X als einzelkettiges Molekül erhalten. Das einzelkettige Faktor X-Molekül zeichnet sich durch seine hohe Stabilität und molekulare Integrität aus. Bisher konnte ein einzelkettiges Faktor X-Molekül nicht in gereinigter Form isoliert werden, da es sehr rasch in die zweikettige Form prozessiert wird (Fair et al., 1984, Blood 64:194-204). Die rekombinanten einzelkettigen Faktor X-Analoge können durch spezifische Prozessierung in die zweikettige Faktor X-Form prozessiert und anschließend zu Faktor Xa bzw. Faktor Xa-Analogon aktiviert werden. Dies kann dadurch erfolgen, daß ein aus einer Proteasedefizienten Zelle isoliertes einzelkettiges rekombinantes Faktor X-Molekül mit einer bibasischen Protease, wie Furin/PACE oder Kex2 in Kontakt gebracht wird und in zweikettiges Faktor X-Analogon prozessiert wird.

Zweikettiges Faktor X-Analogon kann zu Faktor Xa bzw. Faktor Xa-Analogon aktiviert werden. Dies kann bespielsweise dadurch erfolgen, daß ein Faktor X-Analogon, das durch eine Modifikation in Bereich des Aktivierungspeptids eine Furin-spezifische Spaltstelle aufweist, aus einer Furin-defizienten Zelle als einzelkettiges Molekül isoliert und anschließend durch in Kontakt bringen mit einer Endoprotease in ein aktiviertes Faktor Xa-Molekül prozessiert wird.

Ebenso kann ein isoliertes, einzelkettiges Faktor X-Analogon, das eine Modifikation im Aktivierungspeptid aufweist, die eine alternative Prozessierung durch eine Protease aus der Gruppe der Serinproteasen oder Kallikrein ermöglicht, erst durch Behandlung einer bibasischen Endoprotease, wie etwa Furin, in ein zweikettiges Faktor X-Molekül gespalten und dieses im weiteren Verlauf mit einer Serinprotease derart in Kontakt gebracht werden, daß eine Aktivierung zu Faktor Xa bzw. Faktor Xa-Analogon erfolgt.

Ein als zweikettiges Molekül aus einer Zellkultur isoliertes Faktor X-Analogon kann direkt mit der für die Aktivierung spezifischen Protease behandelt werden.

Ein derart erhaltener Faktor Xa bzw. Faktor Xa-Analogon weist aufgrund der selektiven und gerichteten Prozessierungsreaktion eine hohe Stabilität und strukturelle Integrität auf und ist insbesondere frei von inaktiven Faktor X/Xa-Analogon-Intermediaten und autoproteolytischen Abbauprodukten.

Ein weiterer Aspekt der vorliegenden Erfindung betrifft die rekombinante DNA kodierend für die erfindungsgemäßen Faktor X-Analoge. Die rekombinante DNA resultiert nach Expression in einem Faktor X-Analogon mit einer Aminosäuresequenz entsprechend dem humanen Faktor X, außer daß es eine Modifikation aufweist, die die Prozessierungsspezifität und Prozessierungsprodukte beeinflußt, wogegen die biologische Koagulans-Aktivität im wesentlichen unbeeinflußt bleibt.

Gemäß einem weiteren Aspekt werden auch transformierte Zellen enthaltend die rekombinante DNA zur Verfügung gestellt.

Ein weiterer Aspekt der Erfindung betrifft eine Präparation enthaltend ein gereinigtes Faktor X-Analogon oder ein Vorläuferprotein davon, das eine Modifikation im Bereich der natürlicherweise vorkommenden Faktor Xa-Aktivierungsstelle aufweist. Die Modifikation im Bereich der Aktivierungsspaltstelle ist eine neue, nicht-natürlicherweise an dieser Position im Polypeptid vorkommende Erkennungs- bzw. Spaltstelle für eine Protease, die das Polypeptid normalerweise nicht an dieser Stelle prozessiert. Die Präparation kann dabei eine gereinigte Präparation von einzelkettigem oder zweikettigem Faktor X-Analogon sein, wobei die Polypeptide aus einem Zellkultursystem entweder nach Isolierung aus dem Zellkulturüberstand oder aus einem Extrakt einer Zellkultur erhalten werden. Ein aus einem Zellkultursystem vorgereinigtes rekombinantes Faktor X-Analogon kann über aus dem Stand der Technik bekannte Verfahren weiter gereinigt werden. Dazu eignen sich insbesondere chromatographische Verfahren, wie Gelfiltration, Ionenaustauscher- oder Affinitätschromatographie.

Gemäß einer Ausführungsform enthält die erfindungsgemäße Präpäration das Faktor X-Analogon vorzugsweise als einzelkettiges Molekül in isolierter Form. Eine derartige Präparation wird dadurch hergestellt, daß ein durch rekombinante Herstellung gewonnenes Faktor X-Analogon als einzelkettiges Molekül aus einem Zellsystem, vorzugsweise einer Zellkultur von Zellen, denen die Endoprotease fehlt, welche die Einzelkette in schwere und leichte Kette spaltet.

Gemäß einem besonderen Aspekt enthält die Präparation einzelkettiges Faktor X-Analogon mit einer Modifikation, die eine Aktivierung zu Faktor Xa durch eine der Proteasen, ausgewählt aus der Gruppe der bibasischen Endoproteasen, wie etwa Kexin/Kex2, Furin/PACE, PC1/PC3, PC2, PC4, PACE 4, LPC/PC7 in vitro erlaubt. Die Aktivierung erfolgt dabei durch In-Kontakt-bringen des Faktor X-Analogons mit der Protease, wobei aufgrund der natürlich vorkommenden Prozessierung eine Spaltung in die mature Faktor X-Form und durch die Modifikation eine Abspaltung des Aktivierungspeptids erfolgt und Faktor Xa bzw. Faktor Xa-Analogon entsteht.

In der erfindungsgemäßen Präparation kann das Faktor X-Analogon als einzelkettiges Molekül entweder als Faktor X*α* (FX*α*) oder mit einer Deletion des β-Peptids vorliegen. Die Präparation enthält insbesondere Faktor X-Analogon in enzymatisch inaktiver Form mit einer Reinheit von mindestens 80 %, vorzugsweise 90 %, besonders bevorzugt von 95 %, und enthält keine inaktiven, proteolytischen Intermediate von Faktor X/Xa-Analogon.

Gemäß einer weiteren Ausführungsform enthält die erfindungsgemäße Präparation das Faktor X-Analogon vorzugsweise als zweikettiges Molekül in isolierter Form. Dazu wird beispielsweise ein durch rekombinante Herstellung als einzelkettiges Molekül aus einem Zellsystem gewonnenes Faktor X-Analogon in vitro, also außerhalb der Zelle, durch eine Protease, vorzugsweise eine bibasische Protease, in die zweikettige Form gespalten. Dies kann dadurch erfolgen, daß die Protease direkt mit dem Kulturüberstand der Faktor X-Analogon exprimierenden Klone gemischt wird, entweder durch Mischen der gereinigten Protease oder eines Zellkulturüberstandes einer die Protease in rekombinanter Form exprimierenden Zellkultur, oder durch Co-Kultivierung von Faktor X-Analogon und Protease-exprimierenden Klonen.

Ebenso kann der Zellkulturüberstand, enthaltend das Faktor X-Analogon oder das gereinigte Faktor X-Analogon, mit einer immobilisierten Protease in Kontakt gebracht werden, wobei eine Prozessierung in die zweikettige Form stattfindet. Bei diesem Verfahren ist die Protease vorzugsweise an eine Matrix gebunden und der Zellkulturüberstand oder eine gereinigte Präparation enthaltend das Faktor X-Analogon wird über diese Matrix geleitet. Jedoch ist auch die Immobilisierung des Faktor X-Analogons denkbar, während die Protease in der mobilen Phase ist. Ebenso können die Reaktanden (Faktor X-Analogon und Protease) gemischt und über einen bestimmten Zeitraum inkubiert werden. Die Protease wird anschließend aus dem Gemisch, beispielsweise durch Affinitätschromatographie entfernt.

Die zweikettige Form des Faktor X-Analogons kann auch durch Co-expression der Protease und des Faktor X-Analogon direkt in einer gegebenen Zelle gewonnen und gegebenenfalls gereinigt werden.

Gemäß einer besonderen Ausführungsform der Erfindung enthält die Präparation ein einzelkettiges oder zweikettiges Faktor X-Analogon mit einer Modifikation, die eine Aktivierung zu Faktor Xa bzw. Faktor Xa-Analogon in vitro erlaubt. Die Aktivierung von Faktor X-Analogon zu Faktor Xa bzw. Faktor Xa-Analogon kann dabei erfolgen durch In-Kontakt-bringen des Faktor X-Analogons mit einer Protease, ausgewählt aus der Gruppe der bibasischen Endoproteasen, wie etwa Kexin/Kex2, Furin/PACE, PC1/PC3, PC2, PC4, PACE 4, LPC/PC7, der Gruppe der Serinproteasen, wie etwa Faktor XIIa, Faktor XIa, Faktor IIa, Faktor Xa, oder von Kallikrein, oder einem Derivat dieser Proteasen. Die Protease kann dabei an einen Träger immobilisiert sein.

Die erfindungsgemäße Präparation kann als Ausgangsmaterial zur Herstellung und Gewinnung von Faktor Xa dienen. Dazu wird in einem großtechnischen Ansatz die Präparation, enthaltend einzelkettiges oder zweikettiges Faktor X-Analogon, beispielsweise mit einer, gegebenenfalls immobilisierten, Protease unter Bedingungen, die eine optimale Aktivierung von Faktor X-Analogon zu Faktor Xa erlauben, in Kontakt gebracht und Faktor Xa bzw. Faktor Xa-Analogon erhalten. Der so gewonnene Faktor Xa/-Analogon kann anschließend über allgemein bekannte Methoden zu einer pharmazeutischen Zusammensetzung formuliert werden.

Gemäß einer besonderen Ausführung enthält die Präparation, enthaltend das gereinigte, einzelkettige oder zweikettige Faktor X-Analogon, einen physiologisch akzeptablen Träger und ist gegebenenfalls als pharmazeutisches Präparat formuliert. Die Formulierung kann in an sich üblicher Weise erfolgen und mit einem Puffer enthaltend Salze, wie NaCl, CaCl₂, und Aminosäuren, wie Glycin und/oder Lysin, bei einem pH im Bereich von 6 bis 8 gemischt und als pharmazeutisches Präparat formuliert sein. Die gereinigte Präparation enthaltend Faktor X-Analogon kann als fertige Lösung, Lyophilisat oder tiefgefroren bis zum Endgebrauch als lagerfähiges Produkt bereitgestellt werden. Vorzugsweise erfolgt die Lagerung der Präparation in lyophilisierter Form und wird mit einer entsprechenden Rekonstitutionslösung in eine optisch klare Lösung gelöst.

Die Präparation gemäß der vorliegende Erfindung kann jedoch auch als Flüssigpräparat bzw. in flüssig-tiefgefrorener Form zur Verfügung gestellt werden. Die erfindungsgemäße Präparation ist besonders stabil, d.h. sie kann auch in gelöster Form über längere Zeit vor der Applikation stehen gelassen werden. Es hat sich gezeigt, daß die erfindungsgemäße Präparation für mehrere Stunden bis Tage keinerlei Aktivitätsverlust zeigt.

Die erfindungsgemäße Präparation kann in einer geeigneten Vorrichtung, vorzugsweise einer Applikationsvorrichtung, in Kombination mit einer Protease, ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor IIa, Faktor XIIa, Faktor XIa, Faktor Xa, oder von Kallikrein, oder einem Derivat dieser Proteasen, vorliegen.

Die erfindungsgemäße Präparation enthaltend ein Faktor X-Analogon in Kombination mit einer Protease, die in der Lage ist, das Faktor X-Analogon zu Faktor Xa bzw. Faktor Xa-Analogon zu aktivieren, kann als Kombinationspräparat bereitgestellt werden, bestehend aus einem Behälter enthaltend eine an einen Träger immobilisierte Protease, gegebenenfalls in Form einer Mini-Säule oder einer mit einer Protease bestückten Spritze und einem Behälter enthaltend die pharmazeutische Präparation mit Faktor X-Analogon. Zur Aktivierung des Faktor X-Analogons wird die Faktor X-Analogon-haltige Lösung beispielsweise über die immobilisierte Protease gedrückt. Die Faktor X-Analogon-haltige Lösung ist dabei während der Lagerung des Präparates vorzugsweise von der Protease räumlich getrennt. Die erfindungsgemäße Präparation kann im gleichen Behälter wie die Protease sein, wobei die Komponenten jedoch durch eine impermeable Trennwand, die bei etwaigem Gebrauch leicht zu entfernen ist, räumlich getrennt sind. Die Lösungen können auch in eigenen Behältern aufbewahrt werden und erst kurz vor Anwendung miteinander in Kontakt geracht werden.

In einer besonderen Ausführungsform ist die zur Aktivierung eingesetzte Protease eine auch natürlicherweise bei der Blutgerinnung beteiligte Serinprotease, wie etwa Faktor XIIa oder Faktor XIa, die vor der Applikation nicht vom aktivierten Faktor Xa abgetrennt werden muß, sondern mit ihr appliziert werden kann.

Die Aktivierung von Faktor X-Analogon zu Faktor Xa kann kurz vor dem direkten Gebrauch, also vor der Applikation am Patienten erfolgen. Die Aktivierung kann durch in Kontakt-bringen mit einer immobilisierten Protease oder durch Mischen von Lösungen, enthaltend einerseits eine Protease und Faktor X-Analogon andererseits, erfolgen. Es ist daher möglich die beiden Komponenten getrennt voneinander in Lösung zu halten und durch eine geeignete Infusionsvorrichtung, bei der die Komponenten während des Durchlaufs in Kontakt kommen, zu mischen und so das jeweilige Molekül zu Faktor Xa bzw. Faktor Xa-Analogon zu aktivieren. Dem Patienten wird so ein Gemisch von Faktor Xa und einer weiteren Serinprotease, die die Aktivierung bewirkt hat, verabreicht. Hierbei ist insbesondere auf die Dosierung zu achten, da durch die zusätzliche Gabe einer Serinprotease auch endogener Faktor X aktiviert wird und damit die Gerinnungszeit verkürzt sein kann.

Gemäß einer bevorzugten Ausführungsform wird die pharmazeutische Präparation in einer geeigneten Vorrichtung, vorzugsweise einer Applikationsvorrichtung, entweder in flüssig-gefrorener oder lyophilisierter Form zur Verfügung gestellt. Eine geeignete Applikationsvorrichtung kann dabei ein gemäß der AT 366 916 oder der AT 382 783 beschriebener Doppelkammerspritzenkörper sein.

Gemäß einen Aspekt der Erfindung enthält die Präparation ein Faktor X-Analogon mit einer Modifikation, die Aktivierung von Faktor X-Analogon zu einem Faktor Xa in vivo erlaubt. Die Faktor X-Analoge der erfindungsgemäßen Präparation weisen insbesondere eine Modifikation auf, die eine Erkennungsstelle/Spaltstelle für eine Protease, ausgewählt aus der Gruppe der Serinproteasen, wie etwa Faktor XIIa, Faktor XIa, Faktor Xa, oder von Kallikrein darstellt und werden durch eine der genannten Proteasen in vivo zu nativem Faktor Xa bzw. Faktor Xa-Analogon gespalten. Dabei sind besonders für den therapeutischen Einsatz solche Faktor X-Analoge von Vorteil, die eine Erkennungs-/Spaltstelle für eine innerhalb der Gerinnungskaskade vom Faktor VIIa/Gewebe-Komplex und dem Tenase-Komplex unabhängige Protease aufweisen. Dadurch kann die erfindungsgemäße Präparation bei der Blutstillung sowohl bei Patienten mit Defizienzen des Faktor IX, des Faktor VII als auch des Faktor VIII eingesetzt werden. Bei Patienten, bei denen eine Störung der Blutgerinnung aufgrund einer Faktor XI- oder Faktor XII-Defizienz auftritt, sollten keine pharmazeutischen Präparationen enthaltend Faktor X-Analogon, die durch Faktor XIIa oder Faktor XIa aktiviert werden, eingesetzt werden. Bei z.B. Faktor XI-Defizienz wäre der Einsatz eines Faktor X-Analogon mit einer Faktor XIIa-Schnittstelle einsetzbar.

Gemäß einem weiteren Aspekt der Erfindung enthält die erfindungsgemäße Präparation gegebenenfalls als weiteren Bestandteil einen Blutfaktor in Form des Zymogens oder einer aktiven Serinprotease. Bevorzugt sind dabei als weitere Bestandteile solche Komponenten mit Faktor VIII-Bypass-Aktivität. Dazu gehören insbesondere Faktor II, Faktor VII, Faktor IX, Faktor VIII, Faktor V und/oder die aktiven Serinproteasen davon. Weitere Bestandteile können jedoch auch Phospholipide, Ca-Ionen u.a. sein. Gemäß einer besonderen Ausführungsform der Erfindung enthält die erfindungsgemäße Präparation mindestens eine weitere Komponente mit Faktor VIII-Bypass-Aktivität.

Die erfindungsgemäße Präparation kann als pharmazeutische Präparation mit Faktor Xa-Aktivität als Einkomponenten-Präparat oder in Kombination mit anderen Faktoren als Mehrkomponentenpräparat zur Verfügung gestellt werden.

Vor der Aufbereitung in eine pharmazeutische Präparation wird das gereinigte Protein den üblichen Qualitätskontrollen unterzogen und in eine therapeutisch verabreichbare Form gebracht. Insbesondere wird bei der rekombinanten Herstellung das gereinigte Präparat auf Abwesenheit von zellulären und vom Expressionsvektor stammenden Nukleinsäuren getestet, vorzugsweise gemäß einem Verfahren, wie es in der EP 0 714 987 beschrieben ist.

Da prinzipiell jedes biologische Material mit infektiösen Keimen kontaminiert sein kann, wird zur Herstellung eines sicheren Präparates die Präparation gegebenenfalls zur Inaktivierung bzw. Abreicherung von Viren behandelt.

Gemäß einem weiteren Aspekt der vorliegenden Erfindung wird eine Präparation, enthaltend Faktor Xa-Analogon mit hoher Stabilität und struktureller Integrität, zur Verfügung gestellt, die insbesondere frei ist von inaktiven Faktor X/Xa-Analogon-Intermediaten und autoproteolytischen Abbauprodukten und dadurch erhältlich ist, daß ein Faktor X-Analogon der oben beschriebenen Art aktiviert und zu einer entsprechenden Präparation zubereitet wird.

Ein weiterer Aspekt der Erfindung betrifft die Verwendung einer Präparation der oben beschriebenen Art zur Herstellung eines Arzneimittels. Ein Arzneimittel enthaltend ein erfindungsgemäßes Faktor X-Analogon bzw. Faktor Xa-Analogon eignet sich insbesondere zur Behandlung von Patienten mit Störungen der Blutgerinnung, wie etwa Hämophilie-Patienten oder Patienten, welche inhibierende Antikörper gegen die üblicherweise zur Behandlung eingesetzten Faktor VIII und/oder IX entwickelt haben, und insbesondere als Präparat mit Faktor VIII-Bypass-Aktivität.

Ein weiterer Aspekt der Erfindung betrifft die Verwendung einer Nukleinsäure enthaltend die kodierenden Sequenzen der erfindungsgemäßen Faktor X-Analogen zur Herstellung eines Arzneimittels. Dabei kann die Nukleinsäure, sofern sie geeignete Expressionskontrollsequenzen enthält, als nackte Nukleinsäure appliziert werden, in einem rekombinanten Expressionsvektor integriert sein oder an einen Träger, entweder ein Phospholipid oder ein virales Partikel gebunden sein. Die Nukleinsäure kann dabei zur Herstellung eines Arzneimittels verwendet werden, das sich insbesondere zur Behandlung von Patienten mit Störungen der Blutgerinnung, wie etwa Hämophilie-Patienten oder Hämophilie-Patienten mit inhibitorischen Antikörpern eignet. Ein Einsatz der Nukleinsäure in der Gentherapie ist ebenfalls denkbar.

Ein weiterer Aspekt der Erfindung betrifft ein Verfahren zur Herstellung der erfindungsgemäßen Faktor X-Analogen und eine Präparation enthaltend ein erfindungsgemäßes Faktor X-Analogon. Dazu wird eine für ein Faktor X-Analogon kodierende Sequenz in ein geeignetes Expressionsystem eingebracht und entsprechende Zellen, vorzugsweise permanente Zellinien, mit der rekombinanten DNA transfiziert. Die Zellen werden unter optimalen Bedingungen für die Genexpression kultiviert und Faktor X-Analoge entweder aus dem Extrakt einer Zellkultur oder dem Zellkulturüberstand isoliert. Die Reinigung des rekombinanten Moleküls kann durch alle bekannten chromatographischen Verfahren, wie Anionen- oder Kationenaustauscher-, Affinitäts- oder Immunaffinitätschromatographie, oder einer Kombination davon, weiter gereinigt werden.

Zur Herstellung der erfindungsgemäßen Faktor X-Analogen wird die komplette für Faktor X kodierende cDNA in einen Expressionsvektor kloniert. Dies erfolgt entsprechend den allgemein bekannten Klonierungstechniken. Die für Faktor X kodierende Nukleotidsequenz wird anschließend derart modifiziert, daß die kodierende Sequenz im Bereich des Aktivierungspeptids und gegebenfalls auch im Bereich des C-terminalen β-Peptids derart verändert wird, daß ein Faktor X-Molekül der oben beschrieben Art hergestellt werden kann. Dies erfolgt durch aus dem Stand der Technik bekannte gentechnische Methoden, wie spezifische gerichtete in vitro-Mutagenese, oder Deletion von Sequenzen, beispielsweise durch Restriktionsverdau durch Endonukleasen und Insertion anderer, veränderter Sequenzen, oder durch PCR. Die so hergestellten Faktor X-Mutanten werden dann in ein für die rekombinante Expression geeignetes Expressionssystem inseriert und exprimiert.

Die erfindungsgemäßen Faktor X-Analoge können ebenfalls durch chemische Synthese hergestellt werden.

Die Faktor X-Analoge werden vorzugsweise durch rekombinante Expression hergestellt. Die gentechnische Herstellung kann mit allen gängigen Expressionssystemen, wie z.B. permanenten Zelllinien oder viralen Expressionssystemen, erfolgen. Die permanenten Zellinien werden hergestellt durch stabile Integration der Fremd-DNA in das Wirtszellchromosom von z.B. Vero, MRC5, CHO, BHK, 293, Sk-Hepl, insbesondere Leber- und Nierenzellen, oder durch einen episomalen Vektor, abgeleitet von z.B. Papilloma Virus. Virale Expressionssysteme, wie beispielsweise Vaccinia Virus, Baculovirus oder retrovirale Systeme können ebenfalls eingesetzt werden. Als Zellinien werden allgemein Vero, MRC5, CHO, BHK, 293, Sk-Hep1, Drüsen-, Leber- und Nierenzellen eingesetzt. Als eukaryontische Expressionssysteme können auch Hefen, endogene Drüsen (z.B. Drüsen transgener Tiere) und andere Zelltypen verwendet werden. Natürlich können auch transgene Tiere zur Expression der erfindungsgemäßen Polypeptide oder Derivaten davon verwendet werden. Zur Expression der rekombinanten Proteine haben sich im speziellen CHO-DHFR-Zellen bewährt (Urlaub et al., 1980, Proc. Natl. Acad. Sci., USA, 77:4216-4220).

Zur rekombinanten Herstellung der erfindungsgemäßen Faktor X-Analoge können auch prokaryontische Expressionssysteme eingesetzt werden. Hierzu eignen sich insbesondere Systeme, die eine Expression in E. coli oder B. subtilis erlauben.

Die Faktor X-Analoge werden in den entsprechenden Expressionssystemen unter der Kontrolle eines geeigneten Promotors exprimiert. Im Fall der Expression in Eukaryonten eignen sich dazu alle bekannten Promotoren, wie SV40-, CMV-, RSV-, HSV-, EBV-, ß-Actin-, hGH oder induzierbare Promotoren, wie z.B. hsp- oder Metallothionein-Promotor. Vorzugsweise werden die Faktor X-Analoge unter Kontrolle des β-Actin-Promotors in CHO-Zellen exprimiert.

Gemäß einer Ausführungsform der Erfindung umfaßt das Verfahren zur Herstellung der erfindungsgemäßen Präparation die Schritte des Bereitstellens einer für ein Faktor X-Analogon kodierenden DNA, Transformation einer Zelle mit der rekombinanten DNA, Expression des Faktor X-Analogons, gegebenenfalls in Gegenwart einer Protease, Isolieren des Faktor X-Analogons und gegebenenfalls Reinigung über ein chromatographisches Verfahren.

Gemäß einer Ausführungsform des Verfahrens wird das Faktor X-Analogon als zweikettiges Molekül isoliert. Dazu wird Faktor X-Analogon in einer Zelle exprimiert, die eine Prozessierung von pro-Faktor X-Analogon in zweikettigen Faktor X-Analogon erlaubt. Die Zelle ist dabei vorzugsweise eine Zelle, die eine für die Prozessierung des Faktor X-Vorläufers fähige Protease, etwa eine bibasische Protease, wie Furin oder ein Derivat davon, exprimiert. Gegebenenfalls kann zur Erhöhung bzw. Verbesserung der Prozessierungseffizienz die Zelle derart modifiziert werden, daß sie die Protease verstärkt exprimiert. Dies kann beispielsweise durch Co-Expression einer entsprechenden bibasischen Endoprotease, etwa Furin/PACE, Kex2 oder einem Derivat davon erfolgen. Das erfindungsgemäße Faktor X-Analogon kann ebenfalls in einer Zelle exprimiert werden, die eine normale oder damit für die Prozessierung suboptimale endogene Konzentration einer Protease aufweist und demzufolge eine unvollständige Prozessierung in die zweikettige Form stattfindet. Die anschließende Prozessierung in schwere und leichte Kette erfolgt in diesem Fall, sofern einkettiges Faktor X-Analogon in den Zellüberstand sezerniert wird, wie oben beschrieben, durch Co-Kultivierung mit Protease-exprimierenden Zellen oder In-Kontakt-bringen mit einer, gegebenenfalls immobilisierten, Protease. Der Zellüberstand kann auch über eine Trägermatrix, an die eine Protease gebunden ist, gepumpt werden, wodurch im Eluat zweikettiges Faktor X-Analogon erhalten wird. Auch können die Reaktanden in Lösung gemischt, für einen bestimmten Zeitraum inkubiert und anschließend die Protease beispielsweise über eine Affinitätsmatrix entfernt werden.

Das so erhaltene zweikettige Faktor X-Analogon kann anschließend isoliert, gereinigt und bis zur weiteren Verwendung, wie oben beschrieben, stabil gelagert werden.

In einer besonderen Ausführungsform wird zweikettiges, gegebenenfalls gereinigtes Faktor X-Analogon mit einer Protease ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor XIIa, Faktor XIa, Faktor Xa, Faktor IIa, oder von Kallikrein, oder einem Derivat dieser Proteasen, in vitro Kontakt gebracht wird, unter Bedingungen, unter denen das Faktor X-Analogon zu nativem Faktor Xa oder einem Faktor Xa-Analogon aktiviert wird.

Gemäß einer Ausführungsform erfolgt die Aktivierung durch einen chromatographischen Schritt, bei dem die Protease an einen Träger immobilisiert ist. Gereinigtes zweikettiges Faktor X-Analogon wird dazu über eine Matrix, an die die Protease gebunden ist, geleitet und aus dem Eluat gereinigter Faktor Xa isoliert.

Gemäß einer anderen Ausführungsform werden die Komponenten gemischt und die Protease selektiv aus dem Gemisch entfernt.

Es ist natürlich auch eine Kombination von Prozessierung von einzelkettigem pro-Faktor X-Analogon in die zweikettige Faktor X-Analogon-Form und Aktivierung zu Faktor Xa in einem einzigen Verfahren möglich. Einzelkettiges Faktor X-Analogon oder eine Vorstufe davon wird dazu direkt mit einer bibasischen Protease, vorzugsweise Furin oder einem Derivat davon, die eine Prozessierung in schwere und leichte Kette und eine Aktivierung zu Faktor Xa erlaubt, in Kontakt gebracht. Faktor X-Analogon, das keine Spaltstelle für Furin oder einem Derivat davon im Aktivierungspeptid aufweist, wird gegebenenfalls mit einer weiteren, von der ersten verschiedenen Protease, die jedoch eine Aktivierung ermöglicht, in Kontakt gebracht. Die Proteasen können dabei in einer Mischung, beispielsweise von Furin und Faktor XIa vorliegen.

Die Aktivierung kann auch durch eine Kombination beider Schritte durch nacheinandergeschaltene, miteinander direkt verbundene Vorrichtungen, vorzugsweise von Trägern, wie etwa Säulen, an die die Protease (n) immobilisiert ist (sind), erfolgen. Dabei erfolgt am ersten Träger eine Spaltung von Faktor X in schwere und leichte Kette und am zweiten eine Aktivierung zu Faktor Xa durch die immobilisierte Protease. Eine Kopplung der Träger kann dabei durch direkte Verbindung des Auslaufs der ersten Säule mit dem Einlaß der zweiten Säule erfolgen.

Die Reaktionsbedingungen für die Prozessierungsreaktion(en) und die Aktivierung können ohne weiteres vom Fachmann je nach Versuchsanordnung der gegebenen Rahmenbedingungen optimiert werden. Dabei ist für die Kontaktdauer die Fließgeschwindigkeit der vorliegenden Reaktanden von besonderer Bedeutung. Diese sollte im Idealfall zwischen 0,01 ml/min und 1 ml/min liegen. Als weitere Parameter sind Temperatur, pH-Wert und Elutionsbedingungen von Bedeutung. Nach dem Durchlauf kann aktivierter Faktor Xa gegebenenfalls über selektive Chromatographie weiter gereinigt werden. Die Durchführung des Verfahrens mit jeweils an einen Träger gebundener Protease ist deshalb von besonderem Vorteil, da die Reaktionsanordnung durch Verwendung eines Trägers, vorzugsweise von Chromatographiesäulen, einen zusätzlichen Reinigungsschritt ermöglicht.

Gemäß einem weiteren Aspekt zur Herstellung eines Faktor X-Analogons wird Faktor X-Analogon als einzelkettiges Molekül isoliert. Dazu wird das Faktor X-Analogon in einer Zelle exprimiert, die die Prozessierung der Einzelkette in eine leichte und eine schwere Kette nicht unterstützt. Die Zelle weist dabei vorzugsweise eine Defizienz einer bibasischen Endoprotease, wie etwa Kexin, Furin, PACE auf. Wie im Rahmen der Erfindung festgestellt wurde, ist eine der wesentlichen für die Spaltung von Faktor X in leichte und schwere Kette verantwortlichen Proteasen Furin. Aus einer solchen Endoprotease-defizienten-Mutantenzelle kann Faktor X-Analogon als einzelkettiges Molekül isoliert werden. Ein derart isoliertes und gegebenenfalls gereinigtes Faktor X-Analogon wird anschließend mit einer Protease, ausgewählt aus der Gruppe der Endoproteasen, wie etwa Kexin/Kex2, Furin/PACE, PC1/PC3, PC2, PC4, PACE 4, LPC/PC7, in Kontakt gebracht, unter Bedingungen unter denen einzelkettiges Faktor X-Analogon in die zweikettige Faktor X-Form gespalten wird. Erfindungsgemäße Faktor X-Analoge, die eine Modifikation im Bereich des Aktivierungspeptids aufweisen, die eine Spaltung durch eine dieser Endoproteasen ermöglicht, können durch dieses Verfahren gegebenenfalls direkt durch in Kontakt-bringen mit der Endoprotease von einzelkettigem Faktor X-Analogon zu Faktor Xa bzw. Faktor Xa-Analogon aktiviert werden.

Erfindungsgemäße Faktor X-Analoge, die im Bereich des Aktivierungspeptids eine Modifikation aufweisen, die eine Spaltung durch eine Serinprotease oder Kallikrein ermöglicht, werden nach der Herstellung zu zweikettigem Faktor X-Analogon mit einer weiteren, von der ersten verschiedenen Protease in Kontakt gebracht und zu Faktor Xa-Analogon aktiviert.

Gemäß einem Aspekt der Erfindung wird durch das Verfahren eine Präparation enthaltend aktiven Faktor Xa bzw. ein aktives Faktor Xa-Analogon dadurch erhalten, daß, ein wie oben beschrieben hergestelltes, Faktor X-Analogon einem Aktivierungsschritt unterzogen wird und das aktivierte Polypeptid zu einer gereinigten Präparation, die gegebenenfalls als pharmazeutische Zusammensetzung formuliert ist, weiterverarbeitet wird.

Mit den erfindungsgemäßen Faktor X-Analogen, die durch einen oben beschriebenen Prozeß zu Faktor Xa aktiviert werden, wird gereinigter Faktor Xa bzw. Faktor Xa-Analogon mit hoher Stabilität und struktureller Integrität und insbesondere frei von inaktiven Faktor X/Xa-Intermediaten, gewonnen.

Die Erfindung wird anhand der nachfolgenden Beispiele und der Zeichnungsfiguren näher beschrieben, wobei sie jedoch nicht auf diese besonderen Ausführungsbeispiele beschränkt ist.

Beispiel 1 beschreibt die Konstruktion und Expression eines rFaktor X; Beispiel 2 beschreibt die Prozessierung von rFaktor X in schwere und leichte Kette durch Furin; Beispiel 3 beschreibt die Prozessierung von pro-Faktor X mittels immobilisierter Protease; Beispiel 4 beschreibt die Aktivität des in vitro prozessierten rFaktor X; Beispiel 5 beschreibt die Expression von rFaktor X in Furin-defizienten Zellen; Beispiel 6 beschreibt die Konstruktion und Expression von rFaktor X-Analogen; Beispiel 7 beschreibt die Bestimmung der N-Termini der Faktor X-Prozessierungsprodukte. Beispiel 8 beschreibt die Expression und Charakterisierung des FX-Analogon mit der Furin-Spaltstelle Arg-Arg-Lys-Arg/Ile (rFX^{RRKR/I}) ; Beispiel 9 beschreibt die in vitro-Aktivierung des rFX^{RRKR/I}-Proteins durch rFurin-Derivate; Beispiel 10 beschreibt die Funktionalität des in vitro aktivierten rekombinanten FX-Analogon rFX^{RRKR/I}; Beispiel 11 beschreibt die in vitro-Aktivierung des rFX-Analogon mit der FXIa-Spaltstelle Asp-Phe-Thr-Arg/Val für FXIa.

Es zeigen:
- Figur 1:: Nukleotid-und Aminosäuresequenz von Faktor X
- Figur 2:: Schematische Darstellung der Faktor X-Analogen mit modifizierten Proteasen-Schnittstellen im Bereich des Aktivierungspeptids
- Figur 3:: Schematische Darstellung des Expressionsvektors phAct-rFX
- Figur 4:: Westernblot-Analyse von rFaktor X exprimiert in CHO-Zellen vor und nach Amplifikation
- Figur 5:: Westernblot-Analyse von rFaktor X nach in vitro-Spaltung mit Furinderivaten
- Figur 6:: Westernblot-Analyse von rFaktor X-Molekülen exprimiert in Furin-haltigen und Furin-defizienten Zellen
- Figur 7:: Schematische Darstellung der rFX/rFXa-Analogon-Konstrukte mit veränderten C-Termini der schweren Kette
- Figur 8:: Schematische Darstellung der N-Termini der rFaktor X-Prozessierungsprodukte von Furin-enthaltender und Furin-defizienter CHO-Zellen vor und nach Behandlung mit rFurin
- Figur 9:: Westernblot-Analyse von rFaktor FX^{RRKR/I} , exprimiert in CHO-Zellen
- Figur 10:: Westernblot-Analyse von rFaktor FX^{RRKR/I} nach in vitro-Aktivierung mit Furinderivat.

- Figur 11:: Westernblot-Analyse von rFaktor FX^{DFTR/V} nach in vitro-Aktivierung mit Furinderivat.

Die Expressionsvektoren wurden mittels Standard-Klonierungstechniken (Maniatis et al., "Molecular Cloning"- A Laboratory Manual, Cold Spring Harbor Laboratory, Cold Spring Harbor, New York, USA, 1983) hergestellt. Die Herstellung von DNA-Fragmenten mittels Polymerase-Ketten-Reaktion (PCR) erfolgte durch allgemeine Methoden (Clackson et al., 1991m, PCR A practical approach. ED. McPherson, Quirke, Taylor, S. 187-214).

### Beispiel 1 :

### Expression und Prozessierung von einzelkettigem rFX zu rFX leichte/schwere Kette

### a. Herstellung des rFX Expressionsvektors

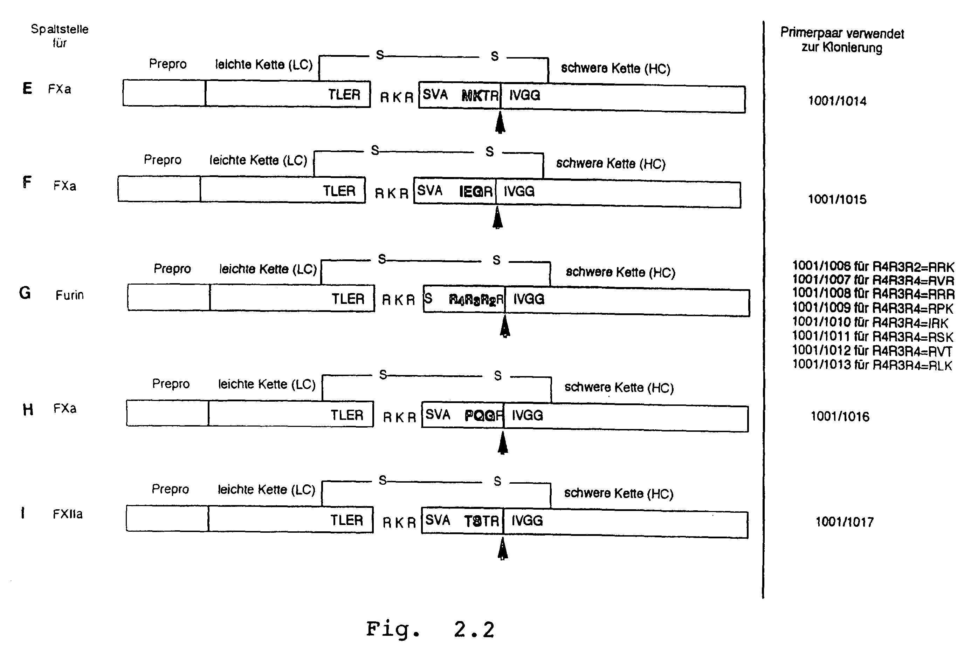

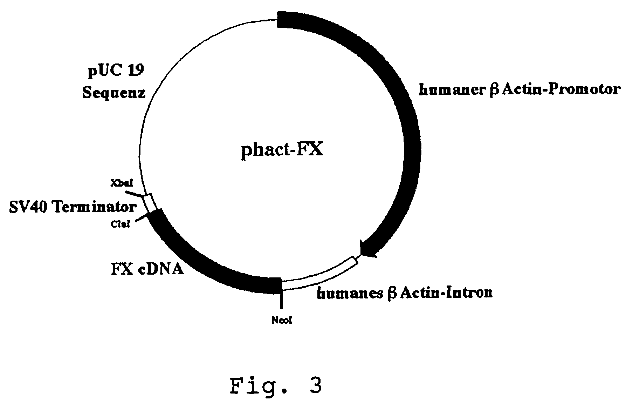

Zur Herstellung von rekombinantem FX (rFX) wurde die cDNA von FX aus einer humanen Leber Lambda-cDNA-Bank, wie von Messier et al. beschrieben (1991, Gene 99:291-294), isoliert. Aus einem positiven Klon wurde mittels PCR mit Oligonukleotid #2911 (5'-ATTACTCGAGAAGCTTACCATGGGGCGCCCACTG-3') (SEQ. ID. Nr. 1) als 5'-Primer und Oligonukleotid #2912 (5'-ATTACAATTGCTGCAGGGATCCAC-3') (SEQ. ID. Nr. 2) als 3'-Primer ein DNA-Fragment amplifiziert, das die 1,467kB FX-kodierende Sequenz sowie 39bp der 3' nichttranslatierten Region, flankiert von einer XhoI-Schnittstelle, am 5'-Ende und einer MfeI-Schnittstelle am 3'-Ende enthält. Zusätzlich wurde durch den Primer #2911 die Sequenz ACC vor das ATG des FX eingebaut, so daß eine optimale Kozak-Translationsinitiations-Sequenz entsteht. Anschließend wurde dieses PCR-Produkt als XhoI/MfeI-Fragment in den mit SalI und EcoRI geschnittenen Expressionsvektor phAct kloniert. Das resultierende Expressionsplasmid wurde mit phAct-rFX bezeichnet (Figur 3). Der Expressionsvektor phAct umfaßt den humanen beta-Actin-Promoter, 78bp 5'UTR sowie das Intron, eine multiple Klonierungsschnittstelle und die SV40-Polyadenylierungsstelle.

### b. Expression von rFX in CHO-Zellen

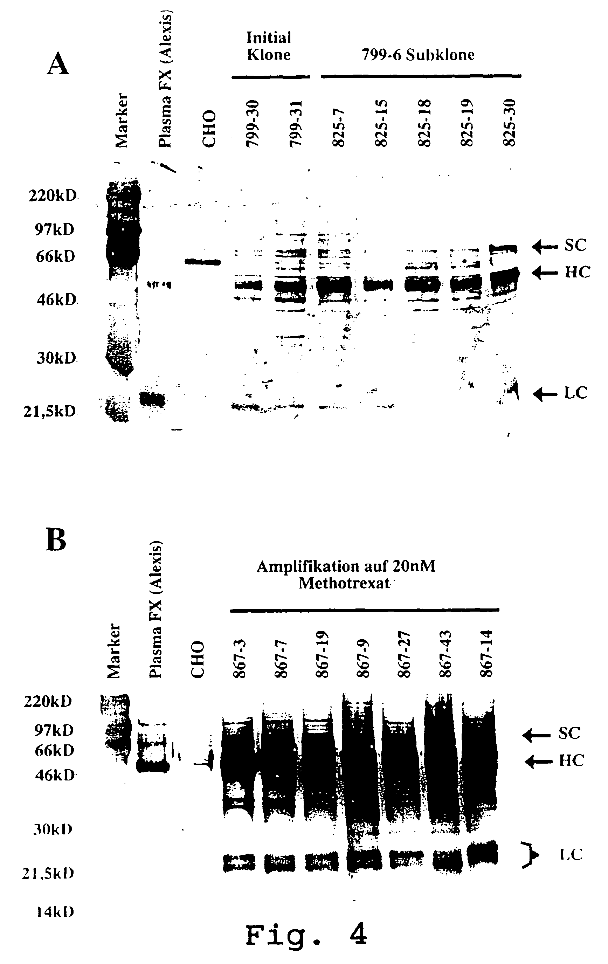

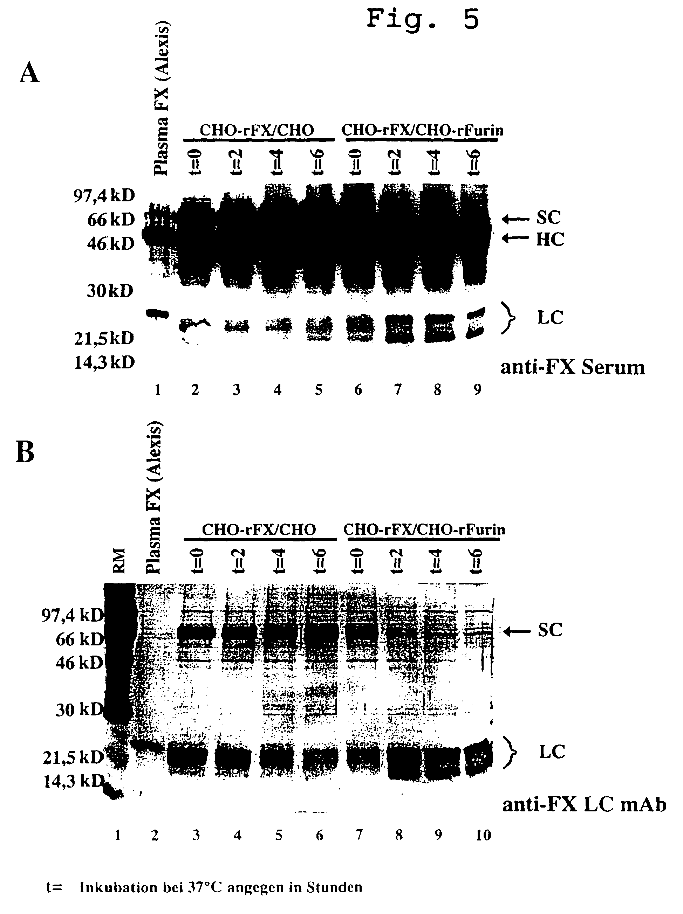

Zur Etablierung einer stabilen rFX-exprimierenden Zellinie wurden dhfr-defiziente CHO-Zellen mit dem Expressionsplasmid phAct-rFX und dem Selektionsmarkerplasmid pSV-dhfr co-transfiziert. Für alle weiteren Expressions- und Funktionsanalysen wurden die Zellkulturen mit serumfreiem Selektionsmedium in Anwesenheit von 10 *µ*g/ml Vitamin K 24 Stunden lang inkubiert. Die Expression von rFX in den resultierenden Zellklonen wurde anhand der Antigenmenge (ELISA, Asserachrom, Boehringer Mannheim) nachgewiesen und das rekombinante Protein anschließend mit SDS-PAGE charakterisiert (Figur 4 A und B). In den Initialklonen und Subklonen davon liegt, wie im Western Blot erkennbar (Figur 4 A), das rekombinante FX-Protein in der Form einer leichten Kette (LC) von 22kD und einer schweren Kette (HC) von ca. 50 kD vor, die identisch mit dem plasmatischen Faktor X-Protein sind. Zusätzlich ist eine Proteinbande bei 75kD zu erkennen, die dem einzelkettigen (SC) Molekül entspricht und deren Präsenz in FX-transfizierten CHO-Zellen (Wolf et al., J. Biol: Chem. 266:13726-13730, 1991) sowie in humanem Plasma (Fair et al., Blood 64:194-204, 1984) beschrieben wurde. Zur Herstellung von hochexprimierenden Klonen wurden die Initialklone mit steigenden Mengen Methotrexat amplifiziert und anschließend bis zur Stabilisierung subkloniert. Die Expression konnte von ca. 200-500 ng/10E6 Zellen bzw. 1 *µ*g/ml auf 78 *µ*g/10E6 Zellen bzw. 120 *µ*g/ml pro 24 Stunden gesteigert werden. Die Western Blot-Analyse dieser hochexprimierenden Zellklonüberstände (Figur 4 B und Figur 5 A Spur 2) zeigt eine Anreicherung des einzelkettigen rFX-Moleküls sowie die Anwesenheit zusätzlicher Formen der leichten Kette. Neben der 22kD-Form der leichten Kette, die der plasmatischen Form entspricht (vollständig carboxyliert und ohne Propeptid) liegen drei weitere Varianten der leichten Kette mit ca. 21kD, 22,5kD und 20kD vor. Die Heterogenität der leichten Kette in diesen Klonen konnte mittels einer N-terminalen Sequenzierung des rekombinanten Materials auf eine unvollständige Abspaltung des Propeptids (hier: ca. 50 % des rFX Materials) sowie auf Untercarboxylierung (hier: ca. 50 % des rFX) zurückgeführt werden. Das 21kD-Protein ist eine untercarboxylierte Propeptid-haltige und das 20kD-Protein eine untercarboxylierte Propeptid-freie Form der leichten Kette, während die 22,5 kD-Bande die vollständig carboxylierte, jedoch Propeptid-haltige LC-Form repräsentiert.

### Beispiel 2 :

### Prozessierung von einzelkettigen rFX in rFX leichte/schwere Kette durch rFurin-Derivate

Aufgrund der Ähnlichkeit der Spaltstellen zwischen Faktor X Propeptid/N-Terminus der leichten Kette (RVTR↓A) und zwischen leichter/schwerer Kette (RRKR↓S) mit der Furin-Konsensus-Erkennungssequenz (RXK/RR↓X) bestand die Möglichkeit, die Prozessierung sowohl einzelkettiger als auch Propeptid-haltiger rFX-Moleküle durch rFurin-Derivate in vitro zu verbessern. In der Literatur werden für die beiden Prozessierungsschritte Proteasen vermutet, bei denen es sich jedoch nicht um Furin handelt (Rehemtulla et al., 1992, Blood 79:2349-2355; Wallin et al., 1994, Thromb. Res. 1994: 395-403).

Zellkulturüberstände von CHO-rFX und CHO-rFurin ΔTM6xHis (Patentanmeldung EP 0 775 750 A2) sowie CHO-rFX und nicht-transfizierter CHO (als Negativkontrolle) wurden im Verhältnis 1:1 gemischt und bei 37°C inkubiert. Aliquote der Reaktionsansätze wurden vor Inkubation (t=0) und nach verschiedenen Inkubationszeiten (t=2, 4, 6 Stunden) mittels Western Blot-Analyse auf prozessierten rFX getestet (Figur 5). Der Nachweis von rFX in den Zellkulturüberständen erfolgte mittels eines anti-humanen FX Antiserums (Figur 5 A) bzw. eines monoklonalen Antikörpers spezifisch für die leichte Kette des FX (Figur 5 B).

Im Gegensatz zu dem CHO-rFX/CHO-Gemisch weist das CHO-rFX/CHOrFurin schon nach zwei Stunden Inkubation bei 37°C (Figur 5 A Spur 7; Figur 5 B Spur 8) eine fast vollständige Prozessierung auf. Einzelkettiger rFX ist zum Großteil in die leichte und schwere Kettenform umgesetzt. Im Bereich der leichten Kette wurden nur noch die prozessierten Propeptid-freien Formen von 22kD (carboxylierte Form) und 20kD (untercarboxylierte Form) in einem Verhältnis von ca. 50:50 gefunden. Durch Optimieren der Zellkulturbedingungen kann dieses Verhältnis zugunsten der carboxylierten Form verbessert werden. Die korrekte Abspaltung der Pro-Sequenz zwischen Arg-1 und Ala+1 und die Homogenität des N-Terminus der leichten Kette wurde mittels N-terminaler Sequenzierung festgestellt. Im Kontrollexperiment, in dem CHO-rFX mit CHO-Überständen gemischt wurde, ist auch nach einer 6-stündigen Inkubation keine Veränderung des rFX-Bandenmusters zu erkennen (Fig. 5A, Spur 5; Fig. 5B, Spur 6). Damit wurde nachgewiesen, daß rFurin im Überstand von CHO-Zellen biologisch aktiv ist und sowohl die Prozessierung des Propeptids als auch der schweren/leichten Kette von rFX durchführen kann.

### Beispiel 3 :

### Prozessierung von Faktor X mittels an Chelat-Tentakelgelimmobilisiertem rFurin

Um festzustellen, ob ein Substrat durch ein Säulen-gebundenes rFurin-Derivat gespalten werden kann, wurde untersucht, ob als Säulen-Matrix statt Ni²⁺-NTA-Agarose in einem experimentellen Ansatz Fractogel EMD®-Tentakelgel (Fa. Merck) verwendet werden kann. Da die Metallionen hierbei im Vergleich zur Ni²⁺-NTA-Agarose räumlich weiter von der eigentlichen Säulen-Matrix entfernt sind, könnte eine verbesserte sterische Zugänglichkeit des Substrats zum gebundenen rFurin-Derivat ermöglicht werden. In dem vorliegenden Ansatz wurde Pro-Faktor X durch Tentakelgel gebundenes rFurin-Derivat prozessiert: Das Fractogel EMD®-Tentakelgel wurde nach Herstellervorschrift mit Ni²⁺-Ionen beladen und mit frischem Serum-freien Zellkulturmedium equilibriert. Anschließend wurde die Säule mit Serum-freiem CHO-rFurin-Derivat-Überstand beladen. Waschschritte erfolgten durch Serum-freies Zellkulturmedium, enthaltend steigende Imidazol-Konzentrationen bis 40mM. Anschließend wurde Pro-Faktor X als Serum-freier CHO-Überstand über die Säule geleitet. Mittels Western Blot-Analyse mit spezifischem Faktor X-Antiserum wurde die Prozessierung von Pro-Faktor X zu zweikettigem Faktor X im Durchfluß der Säule nachgewiesen.

### Beispiel 4 :

### Aktivität des in vitro prozessierten rekombinanten Faktor X

Rekombinanter Faktor X-Vorläufer wurde mit und ohne rFurin bei 4°C inkubiert. Zu verschiedenen Zeiten wurden Proben entnommen und bei -20°C weggefroren. Nach Abschluß der Inkubation (nach 4 Tagen) wurden alle Proben mittels FX-Coatest Kit (Fa. Chromogenix) auf FX-Aktivität getestet. Dazu wurden 50 *µ*l von jedem Überstand mit 50 *µ*l FX-defizientem, humanen Plasma versetzt und laut Protokoll des Herstellers rFX mit Schlangengift (RVV) in Anwesenheit von CaCl₂ zu rFXa umgesetzt; rFXa hydrolysiert anschließend das chromogene Substrat (S-2337) und führt zur Freisetzung des gelbfarbigen Paranitroanilin. Da die Menge an rFXa und die Farbintensität proportional zueinander sind, kann anhand einer Eichgerade, interpoliert aus Werten einer Plasma-Verdünnungsreihe, die Menge zu rFXa aktivierbarem rFX/ml Zellkulturüberstand bestimmt werden. Mit diesen Ergebnissen und der bekannten rFX-Antigenmenge (ELISA-Daten) kann der Anteil von zu Faktor Xa aktiviertem rFaktor X in % ausgerechnet werden. Die Ergebnisse sind in Tabelle 1 dargestellt.

Um unspezifische, proteolytische Aktivität in CHO- und CHO-rFurin-Überständen auszuschließen, wurde das Gemisch dieser beiden Zellkulturüberstände ebenso untersucht.

CHO-rFX inkubiert mit CHO-Überständen (ohne rFurin) als Kontrolle zeigte auch nach 4 Tagen keine wesentliche Änderung der rFXa-Aktivität, die aufgrund der experimentellen Schwankungen bei etwa 800 mU/ml lag und 55 % - 61 % funktionellem rFX entsprach. Wurde im Vergleich dazu CHO-rFX mit CHO-rFurin inkubiert, so entstand während der Inkubationszeit eine konstante Steigerung der rFX-Aktivität, die von etwa 61 % (Zeitpunkt T=O) auf 86 % stieg (Tabelle 1).

Damit wurde nachgewiesen, daß durch in vitro Prozessierung von CHO-rFX aus hochexprimierenden Klonen mittels rFurin-Derivat der Anteil von zu funktionellem rFXa aktivierbaren rFX wesentlich verbessert wird.

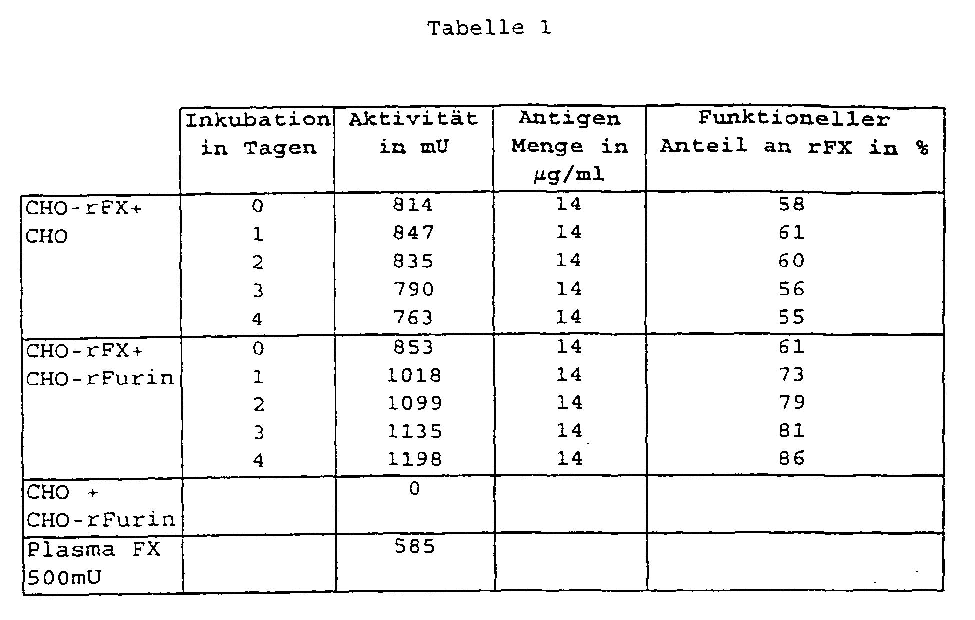

### Beispiel 5 :

### Expression von rekombinantem Faktor X in Furin-defizienten

### Zellen

Wie in den vorangegangenen Beispielen gezeigt, wird bei dem Faktor X-Vorläuferprotein sowohl die Propeptid-Abspaltung als auch die Spaltung der Einzelkette zu leichter/schwerer Kette in vitro durch Furin vermittelt. Dies legt nahe, daß diese Schritte auch in der Zelle endogen durch das ubiquitär vorkommende Furin, abhängig von der jeweilig exprimierten rFaktor X-Menge mit unterschiedlicher Effizienz, bewerkstelligt werden. Dies wiederum führt zur Produktion einer Mischung heterogener rFaktor X-Formen.

Eine Möglichkeit, um eine möglichst homogene und zudem stabile Form von rFaktor X-Molekülen zu erzeugen, ist die Spaltung von rFaktor X durch endogene Proteasen, insbesondere Furin, zu unterbinden und somit funktionell inaktiven rFaktor X-Vorläufer (welcher durch spätere Downstream-Prozessierung, idealerweise direkt vor Verwendung, in seine funktionell aktive Form umgewandelt werden kann) zu produzieren.

Dieses Verfahren wird speziell bei der Erzeugung von FX-Analogon, die eine Furin-Spaltstelle anstatt der ursprünglichen Aktivierungsstelle enthalten, nützlich sein. Bei diesen Konstrukten kann die Aktivierung solch einer rekombinanten rFX-Mutante in vivo durch das endogene Furin stattfinden und zur Sekretion von aktivierten instabileren rFX-Formen führen. Die Degradation dieser Formen, durch CHO-Proteasen, z.B. in Zellkulturbedigungen mit hoher Zelllyse, während der Lagerung der Zellkulturüberstände oder dem Reinigungsverfahren oder durch Autoproteolyse, könnte zu inaktiven Abbauprodukten führen (Wolf et al., 1991).

Dieses Ziel kann z.B. durch Supplementieren des Zellkulturmediums mit Agentien, das die intrazelluläre Furin-Aktivität reduzieren bzw. ausschalten kann, erreicht werden.

Eine andere Möglichkeit ist, a priori Furin-defiziente Zellen zu verwenden (Möhring et al., 1983, Infect. Immun. 41:998-1009; Ohnishi et al., 1994, J. Virol. 68:4075-4079; Gordon et al., 1995, Infect. Immun. 63:82-87).

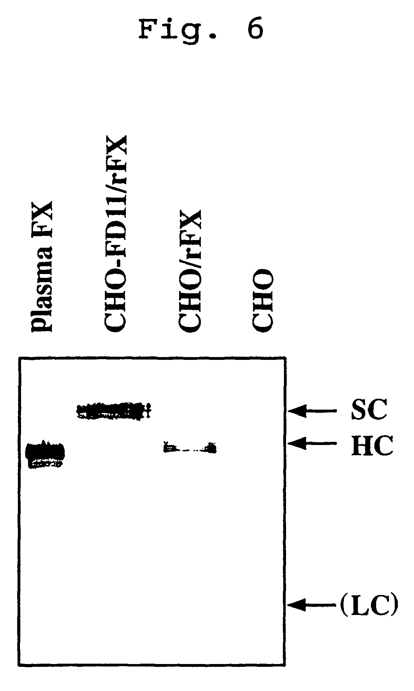

Hierfür wurde ein Furin-defizienter CHO-Zellklon FD11 (Gordon et al., 1995, Infect. Immun. 63:82-87) mit 20 *µ*g phAct-FX und 1 *µ*g pUCSV-neo (enthaltend das Neomycin-Resistenzgen im pUC-Vektor unter der Kontrolle des SV40-Promotors) co-transfiziert. Um stabile Klone zu erhalten, wurde das Medium mit 0,8 *µ*g G418/ml supplementiert. Bei Vergleich sezernierter rFaktor X-Moleküle in serumfreien Überständen eines Furin-haltigen und eines Furin-defizienten CHO-Klons zeigt sich im Westernblot, daß in den Furin-defizienten Zellen rFaktor X-Vorläufer-Prozessierung unterbleibt und nur einzelkettiger Faktor X-Vorläufer vorliegt (Fig. 6); im Gegensatz dazu wird rFaktor X von "normalen" Zellen bei modester Expression noch vollständig, jedoch bei höherer Expression, trotz endogenem Furin, nur noch sehr beschränkt prozessiert. Aufgrund des geringen rFX-Expressionsgrades des verwendeten Zellklons ist die leichte Kette von rFaktor X im Blot hier nicht zu sehen.

### Beispiel 6 :

### Herstellung von Faktor X-Analogen (derzeit nach Ansicht der Anmelderin der beste Weg zur Ausführung der Erfindung)

### 6.1. Konstruktion von Expressionsplasmiden zur Herstellung von Faktor X-Analogon

Zur Herstellung von rekombinanten rFaktor X-Analogon wurde die Spaltstelle Asn-Leu-Thr-Arg/Ile (Aminosäure 231 bis 235) die zur Aktivierung des Faktor X in Faktor Xa dient, durch eine Spaltstelle spezifisch für eine andere Protease, wie Furin, FXIa, FXa, FXIIa, FIIa, oder Kallikrein ersetzt. Die Expressionsplasmide für diese Faktor X-Analogons sind alle vom Plasmid phAct-FX (beschrieben in Beispiel 1) abgeleitet.

Um die Klonierung der Faktor X-Expressionsplasmide zu vereinfachen, wurde das HindIII-NaeI DNA-Fragment aus dem phAct-FX-Expressionsplasmid, das die Faktor X-kodierende Region von Position +1 bis +1116 umfaßt, in die HindIII/SmaI-Restriktionsschnittstellen von Plasmid pUC19 inseriert. Das resultierende Plasmid wurde mit pUC/FX bezeichnet.

Dadurch konnte die Faktor X-Sequenz von Nucleotid an Position 508 bis 705 (Aminosäure 160 bis 235) aus dem pUC/FX-Plasmid leicht entfernt und durch verschieden mutierte Faktor X DNA-Fragmente ersetzt werden. Diese DNA-Fragmente sind mit der deletierten Wildtyp Faktor X-Sequenz identisch, außer den Positionen 691 bis 705 (Aminosäure 231 bis 235), die für neue Spaltstellen kodieren.

Die Wildtyp Faktor X-Sequenz wurde aus dem pUC/FX-Plasmid über Bsp120I- und BstXI-Restriktionsschnitte entfernt. Der 3'-Überhang der BstXI-Stelle wurde zusätzlich mit Mung Bean-Nuclease (Biolab) entfernt.

Die mutierten Faktor X DNA-Fragmente wurden mittels PCR hergestellt. Der 5'-Primer ist identisch für alle Klonierungen und enthält die Faktor X-Sequenz von Position 496 bis 516. Die 3'-Primers enthalten eine Faktor X-komplementierende Sequenz (Position 676 bis 690) und einen nicht-komplementierenden 5'-Überhang, der die Sequenzen für eine neue Spaltstelle und eine Restriktionsschnittstelle trägt. Das amplifizierte PCR-Produkt wurde anschließend mit dem (den) entsprechenden Restriktionsenzym(en) nachgeschnitten und in den vorbereiteten pUC/FX-Vektor (siehe oben) kloniert.

Anschließend wurden die mutierten Faktor X DNA-Fragmente über HindIII-AgeI aus den pUC/FX-Plasmiden in den phAct-FX-Vektor umkloniert. Die Endkonstrukte sind in Figur 2.1 und 2.2 schematisch dargestellt. Als Referenzkonstrukt ist Faktor X-Wildtyp angegeben. Die Aminosäuren sind im Ein-Buchstaben-Code angegeben, die mutierten Positionen sind zusaetzlich schattiert.

Zur Herstellung einer Asp-Phe-Thr-Arg/Val FXIa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1002 (5'-ACCA GTT AAC CCT GGT GAA GTC GTT GTC GCC CCT CTC-3') (SEQ. ID. Nr. 4) benutzt. Daher wurden die Aminosäuren Asn, Leu und Ile an den Positionen 231, 232 und 235 der Faktor X-Sequenz in Asp, Phe und Val ersetzt. Das PCR-Fragment wurde mit Bst120I und HpaI nachgeschnitten (Fig. 2 A).

Zur Herstellung einer Arg/Ser FIIa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1003 (5'-ACCA TCG CGA CCT GGT CAG GTT GTT GTC-3') (SEQ. ID. Nr. 5) benutzt. Daher wurde die Aminosäure Ile an den Positionen 235 in Ser mutiert. Das PCR-Fragment wurde über Bsp120I und NruI nachgeschnitten (Fig.2 B).

Zur Herstellung einer Ile-Lys-Pro-Arg/Ile FXIIa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1004 (5'-ACC AGA ATC GAT TCT GGG TTT GAT GTT GTC GCC CCT CTC-3') (SEQ. ID. Nr. 6) benutzt. Daher wurden die Aminosäuren Asn, Leu und Thr an den Positionen 231, 232 und 233 der FX-Sequenz in Ile, Lys und Pro mutiert. Das PCR-Fragment wurde mit Bst120I und XmnI partiell nachgeschnitten (Fig 2 C).

Zur Herstellung einer Ser-Met-Thr-Arg/Ile Kallikrein-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1005 (5 ' -ACC AGA ATC GAT TCT GGT CAT GCT GTT GTC GCC CCT CTC-3') (SEQ. ID. Nr. 7) benutzt. Daher wurden die Aminosäuren Asn, Leu an den Positionen 231, 232 der Faktor X-Sequenz in Ser, Met mutiert. Das PCR- Fragment wurde mit Bst120I und XmnI partiell nachgeschnitten (Fig. 2 D).

Zur Herstellung einer Pro-Gln-Gly-Arg/Ile FXa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1016 (5'-ACC AGA ATC GAT TCT TCC TTG GGG GTT GTC GCC CCT CTC-3') (SEQ. ID. Nr. 8) benutzt. Daher wurden die Aminosäuren Asn, Leu und Thr an den Positionen 231, 232 und 233 des FX-Proteins in Pro, Gln und Gly mutiert. Das PCR-Fragment wurde mit Bst120I und XmnI partiell nachgeschnitten (Fig. 2 H).

Zur Herstellung einer Met-Lys-Thr-Arg/Ile FXa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1014 (5'-ACC AGA ATC GAT TCT CGT TTT CAT GTT GTC GCC CCT CTC-3') (SEQ. ID. Nr. 9) benutzt. Daher wurden die Aminosäuren Asn, Leu an den Positionen 231, 232 des FX-Proteins in Met, Lys mutiert. Das PCR-Fragment wurde mit Bst120I und XmnI partiell nachgeschnitten (Fig. 2 E).

Zur Herstellung einer Ile-Glu-Gly-Arg/Ile FXa-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1015 (5'-ACC AGA ATC GAT TCT TCC CTC GAT GTT GTC GCC CCT CTC-3') (SEQ. ID. Nr. 10) benutzt. Daher wurden die Aminosäuren Asn, Leu, Thr an den Positionen 231 bis 233 des FX-Proteins in Ile, Glu, Gly mutiert. Das PCR-Fragment wurde mit Bst120I und XmnI partiell nachgeschnitten (Fig. 2 F).

Zur Herstellung einer Arg-Arg-Lys-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1006 (5'-ACC AGA ATC GAT TCT TTT CCT CCT GTT GTC GCC CCT CTC-3') (SEQ. ID. Nr. 11) benutzt. Daher wurden die Aminosäuren Asn, Leu und Thr an Position 231 bis 233 in Arg, Arg und Lys mutiert. Das PCR-Fragment wurde mit Bsp120I und XmnI nachgeschnitten (Fig. 2 G).

Zur Herstellung einer Arg-Val-Arg-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1007 (5'-ACC AGA ATC GAT TCT CCT CAC CCT GTT GTC GCC CCT CTC-3') (SEQ. ID. Nr. 12) benutzt. Daher wurden die Aminosäuren Asn, Leu und Thr an Position 231 bis 233 in Arg, Val und Arg mutiert. Das PCR-Fragment wurde mit Bsp120I und XmnI partiell nachgeschnitten (Fig. 2 G).

Zur Herstellung einer Arg-Arg-Arg-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1008 (5'-ACC AGA ATC GAT TCT CCT CCT CCT GTT GTC GCC CCT CTC-3') (SEQ. ID. Nr. 13) benutzt. Daher wurden die Aminosäuren Asn, Leu und Thr an Position 231 bis 233 in Arg, Arg und Arg mutiert. Das PCR-Fragment wurde mit Bsp120I und XmnI partiell nachgeschnitten (Fig. 2 G).

Zur Herstellung einer Arg-Pro-Lys-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1009 (5'-ACC AGA ATC GAT TCT TTT GGG CCT GTT GTC GCC CCT CTC-3') (SEQ. ID. Nr. 14) benutzt. Daher wurden die Aminosäuren Asn, Leu und Thr an Position 231 bis 233 in Arg, Pro und Lys mutiert. Das PCR-Fragment wurde mit Bsp120I und XmnI partiell nachgeschnitten (Fig. 2 G).

Zur Herstellung einer Ile-Arg-Lys-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1010 (5'-ACC AGA ATC GAT TCT TTT CCT GAT GTT GTC GCC CCT CTC-3') (SEQ. ID. Nr. 15) benutzt. Daher wurden die Aminosäuren Asn, Leu und Thr an Position 231 bis 233 in Ile, Arg und Lys mutiert. Das PCR-Fragment wurde mit Bsp120I und XmnI partiell nachgeschnitten (Fig. 2 G).

Zur Herstellung einer Arg-Ser-Lys-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1011 (5'-ACC AGA ATC GAT TCT TTT GCT CCT GTT GTC GCC CCT CTC-3') (SEQ. ID. Nr. 16) benutzt. Daher wurden die Aminosäuren Asn, Leu und Thr an Position 231 bis 233 in Arg, Ser und Lys mutiert. Das PCR-Fragment wurde mit Bsp120I und XmnI partiell nachgeschnitten (Fig. 2 G).

Zur Herstellung einer Arg-Val-Thr-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1012 (5'-ACC AGA ATC GAT TCT GGT CAC CCT GTT GTC GCC CCT CTC-3') (SEQ. ID. Nr. 17) benutzt. Daher wurden die Aminosäuren Asn, Leu an Position 231, 232 in Arg, Val mutiert. Das PCR-Fragment wurde mit Bsp120I und XmnI partiell nachgeschnitten (Fig. 2 G).

Zur Herstellung einer Arg-Leu-Lys-Arg/Ile Furin-Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1013 (5'-ACC AGA ATC GAT TCT TTT GAG CCT GTT GTC GCC CCT CTC-3') (SEQ. ID. Nr. 18) benutzt. Daher wurden die Aminosäuren Asn und Thr an Position 231 und 233 in Arg und Lys mutiert. Das PCR-Fragment wurde mit Bsp120I und XmnI partiell nachgeschnitten (Fig. 2 G).

Zur Herstellung einer Thr-Ser-Thr-Arg/Ile FXIIa- Spaltstelle wurden als 5'-Primer das Oligonucleotid #1001 (5'-CCC ACA GGG CCC TAC CCC TGT-3') (SEQ. ID. Nr. 3) und als 3'-Primer das Oligonucleotid #1017 (5'-ACC AGA ATC GAT TCT CGT GCT CGT GTT GTC GCC CCT CTC-3') (SEQ. ID. Nr. 19) benutzt. Daher wurden die Aminosäuren Asn, Leu an den Positionen 231, 232 des FX-Proteins in Ile, Lys mutiert. Das PCR-Fragment wurde mit Bst120I und XmnI partiell nachgeschnitten (Fig. 2 I).

### 6.2. Konstruktion von Expressionsplasmiden für die Herstellung von FXβ-Analogon.

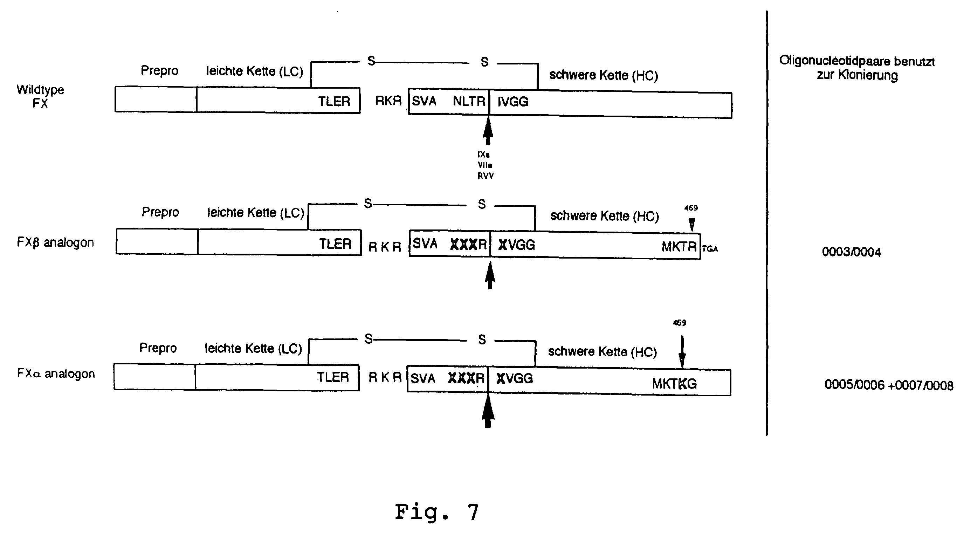

Diese Konstrukte wurden von den oben beschriebenen Faktor X-Analogon-Konstrukten abgeleitet, indem ein TGA-Stopcodon an Position 470 eingebaut wurde. Dazu wurden die Aminosäuren von Position 457 auf cDNA-Level bis zum Stopcodon durch SpeI und partiellen BstEII-Verdau entfernt und mit dem Oligonucleotidpaar #0003 (5'-GTC ACC GCC TTC CTC AAG TGG ATC GAC AGG TCC ATG AAA ACC AGG TGA A-3') (SEQ. ID. Nr. 20) und #0004 (5'-CTA GTT CAC CTG GTT TTC ATG GAC CTG TCG ATC CAC TTG AGG AAG GCG-3') (SEQ. ID. Nr. 21) ersetzt. Eine schematische Darstellung der Faktor Xβ-Analogon-Konstrukte ist in Fig. 7 gezeigt. Zur Vereinfachung der Abbildung wurden alle Faktor Xβ-Analoge als ein allgemeines Konstrukt dargestellt, in dem die variablen Aminosäuren in der Spaltstellenregion als schattiertes "X" angegeben sind.

### 6.3. Konstruktion von Expressionsplamiden für die Herstellung von FXa-Analogon

Mit der Aktivierung des Faktor X durch die Abspaltung des 4,5 kDa-Aktivierungspeptids am N-terminalen Ende der schweren Kette entsteht die Faktor Xaα-Form. Diese Form wird anschließend durch auto-proteolytische Aktivität und Abspaltung des C-Terminus der schweren Kette zwischen Arg 469 und Gly 470 in die FXaβ-Form umgesetzt. Zur Herstellung von Faktor X-Expressionsplasmiden, die zur Produktion von Faktor X führen, die nach Aktivierung ausschließlich als FXaα-Form mit intaktem *β*-Peptid vorliegen, wurde die Aminosäure Arg469 in Lys mutiert, sodaß keine Prozessierung im C-terminalen Bereich der schweren Kette mehr stattfinden kann.

Dazu wurde die C-terminale Aminosäuresequenz des Faktor X von Position 1363 bis zum Stopsignal durch partiellen BstEII-SpeI-Verdau entfernt und durch zwei zusammenligierte Oligonucleotidpaare ersetzt. Oligonucleotid #0005 (5'-GTC ACC GCC TTC CTC AAG TGG ATC GAC AGG TCC ATG AAA ACC AAG GGC TTG CCC AAG-3') (SEQ. ID. Nr. 22) und Oligonucleotid #0006 (5'-TTG GCC TTG GGC AAG CCC TTG GTT TTC ATG GAC CTG TCG ATC CAC TTG AGG AAG GCG-3') (SEQ.

ID. Nr. 23) wurden mit Oligonucleotid #0007 (5'-GCC AAG AGC CAT GCC CCG GAG GTC ATA ACG TCC TCT CCA TTA AAG TGA GAT CCC A-3') (SEQ. ID. Nr. 24) und Oligonucleotid #0008 (5'-CTA GTG GGA TCT CAC TTT AAT GGA GAG GAC GTT ATG ACC TCC GGG GCA TGG CTC-3') (SEQ. ID. Nr. 25) ligiert. Die Mutation der Aminosäure Arg469 wird durch das Oligonucleotidpaar #0005-#0006 eingeführt. Eine schematische Darstellung der FX-Analoge ist in Figur 7 gezeigt.

### Beispiel 7:

### Bestimmung der N-Termini von Faktor X und Prozessierungsprodukten mit und ohne rFurin

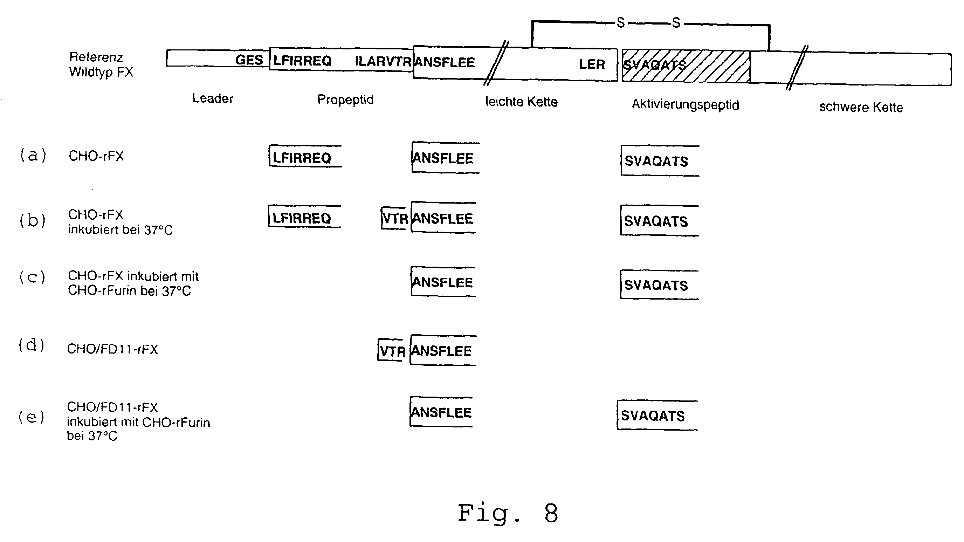

Rekombinanter Faktor X wurde, wie in Beispiel 1 beschrieben, in CHO-Zellen mit endogenem Furin bzw., wie in Beispiel 5 beschrieen, in Furin-defizienten Zellen exprimiert. rFaktor X wurde sowohl aus a) nicht vorbehandeltem, b) weitere 12 Stunden bei 37°C inkubiertem und c) 12 Stunden bei 37°C mit CHO-rFurin-Überstand vorbehandeltem Zellkulturüberstand hochexprimierender CHO-rFX-Klone, als auch aus d) nicht vorbehandeltem und e) 12 Stunden bei 37°C mit CHO-rFurin-Überstand vorbehandeltem Zellkulturüberstand von CHO-FD11-rFX-Klone isoliert. Die N-terminalen Aminosäuren von Faktor X und Prozessierungsprodukten der einzelnen Reaktionsansätze a) bis e) wurden über Edman-Analyse bestimmt. Figur 8 zeigt eine schematische Darstellung der Ergebnisse.

Der rFaktor X aus hochexprimierenden CHO-Zellen tritt in Form der reifen schweren und leichten Ketten, sowie einzelkettig, zum Teil noch Propeptid-haltig auf. Nach einer Inkubation dieser Zellkulturüberstände für 12 Stunden bei 37°C (b) treten zusätzlich, wie schon von Wolf et al. (1991, J. Bio. Chem. 266:13726-13730) beschrieben, fehlerhafte N-Termini der rFX leichten Kette mit 3 zusätzlichen Aminosäuren Va138-Thr39-Arg40 auf. Diese kryptischen Enden werden auch bei der Sequenzierung von rFX-Material aus nicht vorbehandelten CHO-FD11-Zellen (d) gefunden. Diese Beobachtung zeigt, daß das Auftreten von diesen fehlerhaften N-Termini vermieden werden kann, indem optimierte Bedingungen bzw. Zellkulturbedingungen, Lagerung und Reinigungsverfahren zur Minimierung der rFX-Proteolyse durch CHO-Proteasen, benützt werden.

Im Gegensatz zu dem gereinigten Material aus CHO-Zellen (a und b) ist der rFX aus nicht amplifizierten, Furin-defizienten Zellen (d) nur in Form unprozessierter einzelkettiger Vorläufer vorhanden. Es werden auch keine N-terminalen Sequenzen gefunden, die dem Propeptid-Anteil entsprechen. Damit wurde gezeigt, daß die Prozessierung von einzelkettigem rFX-Vorläufer in leichte/schwere Kette in Furin defizienten CHO-Zellen (d) nicht mehr stattfindet, was auf eine zentrale Rolle der Endoprotease Furin in diesem Prozessierungschritt in vivo schliessen läßt.

Zusätzlich wurde gezeigt, daß die Prozessierung Propeptid-haltiger rFX-Moleküle auch in Furin-defizienten CHO-Zellen stattfindet und daher Furin in vivo keine essentielle Rolle in diesem Prozessierungsschritt spielt. Durch die Inkubation von rFX aus CHO-Zellen (c) und CHO-FD11-Zellen (e) in Gegenwart von Furin werden ausschließlich leichte und schwere Ketten mit korrekten N-Termini gefunden. Damit wurde nachgewiesen, daß sowohl die einzelkettigen FX-Vorläufer als auch die Propeptid-haltigen rFX-Moleküle, durch in vitro-Prozessierung in homogenen, reifen Faktor X umgesetzt werden. Damit weist der in Gegenwart von Furin prozessierte Faktor X eine aussergewöhnliche strukturelle Integrität und Homogenität auf.

### Beispiel 8:

### Expression und Charakterisierung des FX-Analogon mit der Furin-Spaltstelle Arg-Arg-Lys-Arg/Ile (rFX^{RRKR/I}).

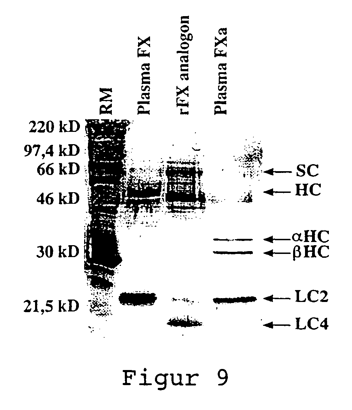

Zur Herstellung von rekombinantem rFx^{RRKR/I}-Protein wurden das FX-Expressionsplasmid mit der Spaltstelle Arg-Arg-Lys-Arg/Ile (siehe Beispiel 6.1, Fig. 2 G) und das Selektionsplasmid pSV/dhfr, wie im Beispiel 1 beschrieben, in CHO-Zellen co-transfiziert. Die Western Blot-Analyse der Zellkultur-Überstände (Figur 9) zeigt, daß das rekombinante Protein vorwiegend in der doppelkettigen Form vorliegt. Im Vergleich zu Plasma FX läuft die schwere Kette bei 46kD anstatt 50kD, was vermutlich auf unterschiedliche Verzuckerungen des rekombinanten Proteins zurückzuführen ist. Zusätzlich sind, wie auch schon bei der Expression von Wild-typ rFX beobachtet (Beispiel 1.b.), geringe Mengen an einzelkettigem Vorläufer (SC) sowie die LC4-Isoform der leichten Kette sichtbar. Diese molekularen Formen des rFX-Analogon weisen auf eine Limitierung sowohl der Prozessierung des einzelkettigen FX-Vorläufers durch endogene Proteasen als auch der γ-carboxylierung der leichten Kette hin. Obwohl die in das FX-Analogon eingefügte Spaltstelle eine Furin-Consensus-Sequenz repräsentiert, sind keine Proteinbanden, die den aktivierten Formen des Proteins entsprächen (35kD, 31kD) sichtbar. Die Struktur im Bereich der Spaltstelle sowie die umgehende Aminosäuresequenz dürften dazu führen, daß die Prozessierung der modifizierten Aktivierungsstelle durch Furin nur suboptimale Bedingungen darstellen.

### Beispiel 9:

### In vitro-Aktivierung des rFX^{RRKR/I}-Proteins durch rFurin-Derivate

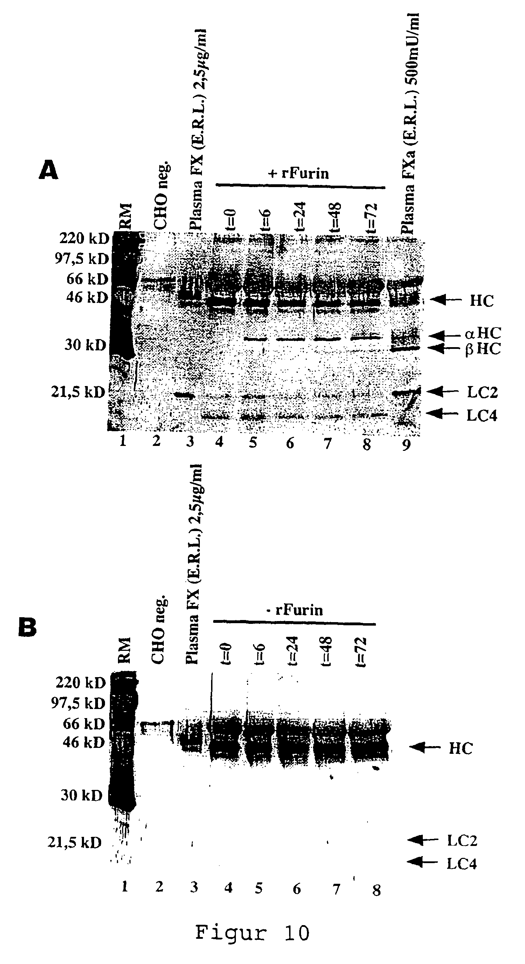

Die Aktivierbarkeit des rekombinanten FX-Analogons in die *α* (35kD) und *β* (31kD) FXa-Formen durch rFurin in vitro wurde, wie in Beispiel 2 für Mischexperimente beschrieben, getestet, mit dem Unterschied, daß gereinigte rFurin-Derivate rFurinΔCys-Spacer-10xHis, beschrieben in der Patentanmeldung EP 0 775 750 A2, in 10mM Hepes, pH 7,0, 150mM NaCl, 2mM CaCl₂ und 0,2% BSA statt CHO-rFurin-Überständen benützt wurden. Im Kontrollexperiment ohne rFurin wurde der CHO-rFX-Analogon-Überstand mit dem Puffer 10mM Hepes, pH 7,0, 150mM NaCl, 2mM CaCl₂ und 0,2% BSA in einem Verhältnis von 1:1 gemischt. Aliquots der Ansätze vor und nach einer Inkubationszeit von 6, 24, 48 und 72 (t=0, 6, 24, 48, 72) Stunden bei 37°C, wurden mittels Western Blot auf rFX-Aktivierung getestet (Fig. 10). Während das Bandenmuster von rFX^{RRKR/I} in Abwesenheit des rFurin-Derivates selbst nach 72 Stunden Inkubation unverändert ist (Fig. 10 B) wird in Anwesenheit von rFurin schon nach 6 Stunden (Fig. 10 A, Spur 5) eine 35kD-schwere Proteinbande, die der α-Form von plasmatischem FX entspricht, sichtbar (Fig. 10 A, Spur 9). Im Laufe der Inkubation akkumuliert diese α-Form und nach 72 Stunden Inkubation (Fig. 10 A, Spur 8) sind etwa 50 % des Ausgangsmaterials (HC) in die aktivierte Form umgesetzt worden. Die zusätzliche 31kD schwere Proteinbande, die nach 24 Stunden (Fig. 10 A, Spur 6) erkennbar wird und der β-Form des aktivierten Plasma FX (Fig. 10 A, Spur 9) entspricht, zeigt, daß die aus rekombinantem FX-Analogon entstandene *α*-Form eine autoproteolytische Aktivität besitzt und daher funktionell ist.

Diese Ergebnisse weisen nach, daß die heterologe Aktivierungsspaltstelle Arg-Arg-Lys-Arg/Ile im rFX-Analogon durch rFurin-Derivate in vitro spezifisch erkannt und korrekt gespalten wird, und somit zur Aktivierung des rFX-Analogon in *α*- und *β*-FXa-Moleküle geeignet ist.

### Beispiel 10:

### Funktionalität des in vitro aktivierten rekombinanten FX-Analogons rFX^{RRKR/I}.

Aliquots der Mischexperimente aus Beispiel 9 wurden mit einem Chromogen-Test auf FXa-Aktivität untersucht. Dazu wurden die Aliquots mit dem Chromogensubstrat S2337 (600*µ*M) in 50mM Tris, pH 7,3, 150mM NaCl, 0,1% BSA versetzt. Nach einer Inkubationszeit von 3 Minuten bei 37°C wurde die Reaktion mit 20% Essigsäure gestoppt und anschließend die OD bei 405nm gemessen. Im Vergleich mit einer Eichgerade, erstellt mittels gereinigtem, RW-aktiviertem Plasma FXa, wurde die Menge an rekombinanter FXa-Aktivität in den Reaktionsansätzen bestimmt. Die Ergebnisse dieser Analyse, die eingesetzten Antigenmengen (ELISA-Werte), sowie die daraus berechnete spezifische Aktivität sind in Tabelle 2 dargestellt.

Um unspezifische amidolytische Aktivitäten in der rFurin-Lösung und dem CHO-Zellkulturüberstand auszuschließen, wurde auch das Gemisch eines Überstandes nicht transfizierter CHO-Zellen mit gereinigtem rFurin-Derivat auf FXa-Aktivität (CHO+rFurin) untersucht. In diesem Gemisch, genau wie im rFX-Analogon/Puffer-Gemisch (rFX^{RRKR/I}+Puffer), konnte auch nach 72 Stunden Inkubation keine FXa-Aktivität gemessen werden. Im Gegensatz dazu war bei der rFX^{RRKR/I}/rFurin-Reaktion bereits nach 6 Stunden Inkubation eine rFXa-Aktivität von 56mU meßbar, die konstant im Laufe der Inkubation anstieg und nach 72 Stunden 133mU erreichte. Obwohl zu diesem Zeitpunkt, laut Western Blot, nur etwa die Hälfte des rFX-Analogons in die aktivierten *α*- und *β*-Formen umgesetzt worden war (Fig.10 A, Spur 8), erreichte das in vitro aktivierte rFX-Analogon-Material mit 190mU/µg eine weit höhere spezifische Aktivität als der mit RVV vollständig aktivierte Plasma FX (153mU/µg). Die gemessenen Aktivitätssteigerungen spiegeln sich im Auftreten der *α*- und *β*-Formen im Western Blot wider (Fig. 10 A, Spur 5-8).

Damit wird nachgewiesen, daß in FX heterologe Protease-Schnittstellen eingebaut werden können, letztere von der zugehörigen Protease erkannt und gespalten werden, und hochwertiger rFXa (oder ggf. rFXa-Analoge mit FXa-Aktivität) in funktioneller Form rekombinant hergestellt werden kann.

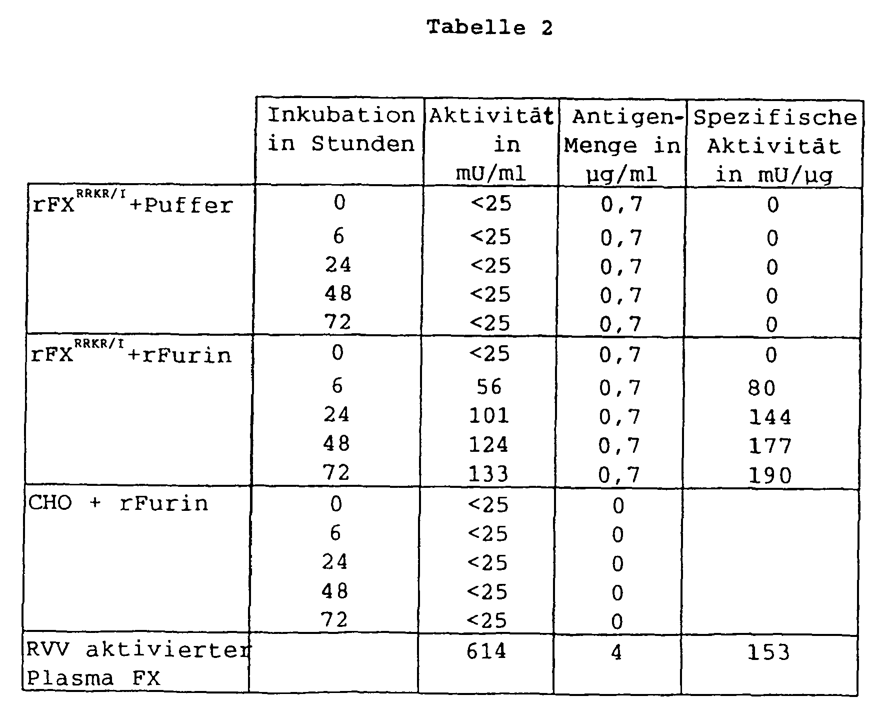

### Beispiel 11:

### In vitro-Aktivierung von rFX-Analogon mit der FXIa-Spaltstelle Asp-Phe-Thr-Arg/Val (rFX^{DFTR/V}) durch plasmatischen FXIa.

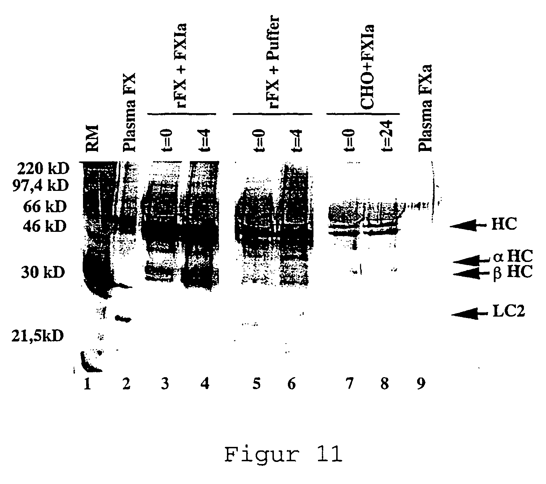

Durch Mutagenese der FX-Aktivierungssequenz in eine FXIa-Spaltstelle wurde ein FX-Analogon-Konstrukt hergestellt. Anschliessend wurden stabile CHO-Zellklone erstellt, die diese Moleküle exprimieren. CHO-Zellkulturüberstand mit rFX^{DFTR/V} wurden mit gereinigtem plasmatischen FXIa (100µg/ml) in Anwesenheit von 10mM Tris, pH 7,3, 150mM NaCl, 8mM CaCl₂, PCPS und 0,1% BSA gemischt und auf 37°C für verschiedene Zeiten inkubiert. Als Negativkontrolle wurde der Zellkulturüberstand nur mit dem BSA-haltigen Puffer inkubiert. Das rFX^{DFTR/V}-Protein und die resultierenden Aktivierungsprodukte wurden mittels Western Blot-Analyse charakterisiert (Figur 11). Wie im Gemisch ohne FXIa vor der Inkubation (t=0) erkennbar ist, wird das rekombinante Protein (Fig. 11, Spur 5) fast identisch zum plasmatischen FX (Spur 2) in der doppelkettigen Form sekretiert mit dem Unterschied, daß die schwere Kette (HC), wie auch schon beim rFX^{RRKR} aus dem Beispiel 8 beobachtet, ein geringfügig kleineres Molekulargewicht als 50kD hat. Während der Inkubation dieses Gemisches bei 37°C (Fig. 11, Spur 6) ist keine wesentliche Änderung des Bandenmusters sichtbar. Im CHO-rFX/FXIa-Gemisch sind, schon kurz nach der Zugabe von gereinigtem FXIa aber vor der eigentlichen Inkubation des Zellkulturüberstandes (Fig. 11, Spur 3), Proteinbanden von 35kD und 31kD zu erkennen, die der Größe der plasmatischen *α*- und *β*-Formen der schweren Kette (Fig. 11, Spur 9) entsprechen. Diese beiden Formen nehmen nach 4 stündiger Inkubation mit FXIa stark zu (Fig. 11, Spur 4).

Hierdurch wurde gezeigt, daß ein FX-Analogon, das die heterologe Proteaseschnittstelle für ein in der Gerinnungskaskade aktives proteolytisches Enzym trägt, vom letzteren auch erfolgreich prozessiert werden kann.

Zusätzlich wird durch das Auftreten der rFXaβ-Bande, dem Ergebnis der autoproteolytischen Aktivität des rFXaα-Analgon, erfolgreich die funktionelle Aktivität des resultierenden rFXaα-Analogen gezeigt.

## Patentansprüche

1. Faktor X-Analogon, **dadurch gekennzeichnet, dass** es eine Modifikation im Bereich der natürlicherweise vorkommenden Faktor Xa-Aktivierungsspaltstelle mit einer Faktor X-Sequenz Gly228-R6-R5-R4-R3-R2-Arg234-R1, bezogen auf die Aminosäurenumerierung gemäß Fig . 1, aufweist, wobei die Modifikation eine Prozessierungsstelle einer nicht-natürlicherweise in diesem Bereich der Faktor X-Sequenz spaltenden Protease darstellt und
a) R1 eine Aminosäure ausgewählt aus der Gruppe Ile, Val, Ser, Thr oder Ala
b) R2 eine Aminosäure ausgewählt aus der Gruppe Pro, Gly, Lys oder Arg
c) R3 eine Aminosäure ausgewählt aus der Gruppe Phe, Lys, Met, Gln, Glu, Ser, Val, Arg oder Pro
d) R4 eine Aminosäure ausgewählt aus der Gruppe Asp, Ile, Ser, Met, Pro, Thr, Arg oder Lys
e) R5 eine Aminosäure ausgewählt aus der Gruppe Asn, Lys, Ser, Glu, Ala, Gln, His oder Arg und
f) R6 eine Aminosäure ausgewählt aus der Gruppe Asp, Phe, Thr, Arg, Leu oder Ser ist.

2. Faktor X-Analogon nach Anspruch 1, **dadurch gekennzeichnet, dass** die Modifikation mindestens eine Aminosäure innerhalb der Aminosäuresequenz des Aktivierungspeptids betrifft.

3. Faktor X-Analogon nach einem der Ansprüche 1 oder 2, **dadurch gekennzeichnet, dass** die Modifikation einen Austausch mindestens einer der Aminosäuren zwischen Gly228 und Arg234, und gegebenenfalls Ile235, bezogen auf die Aminosäurenumerierung gemäß Fig. 1, darstellt.

4. Faktor X-Analogon nach einem der Ansprüche 1 bis 3, **dadurch gekennzeichnet, dass** die Modifikation eine Prozessierungsstelle für eine Protease, ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor XIIa, Faktor XIa, Faktor Xa oder von Kallikrein, oder einem Derivat dieser Proteasen, darstellt.

5. Faktor X-Analogon nach einem der Ansprüche 1 bis 4, **dadurch gekennzeichnet, dass** es eine weitere Modifikation im Bereich der C-terminalen Faktor X-Aminosäuresequenz aufweist.

6. Faktor X-Analogon nach Anspruch 5, **dadurch gekennzeichnet, dass** es eine Modifikation im C-terminalen Bereich der β-Peptidspaltstelle aufweist.

7. Faktor X-Analogon nach Anspruch 6, **dadurch gekennzeichnet, dass** die Modifikation eine Mutation, Deletion oder Insertion im Bereich der Faktor X-Aminosäuresequenz zwischen Aminosäureposition Arg469 und Ser476 ist.

8. Faktor X-Analogon nach einem der Ansprüche 5 bis 7, **dadurch gekennzeichnet, dass** die Modifikation eine Abspaltung des β-Peptids verhindert.

9. Faktor X-Analogon nach Anspruch 5, **dadurch gekennzeichnet, dass** es eine Deletion des Faktor X β-Peptids aufweist.

10. Faktor X-Analogon nach Anspruch 5, **dadurch gekennzeichnet, dass** es ein Translationsstopsignal im C-terminalen Bereich der Faktor X-Sequenz aufweist -

11. Faktor X-Analogon nach Anspruch 10, **dadurch gekennzeichnet, dass** es ein Translationsstopsignal an der Position der Aminosäure 470 der Faktor X-Sequenz aufweist.

12. Faktor X-Analogon nach einem der Ansprüche 1 bis 11, **dadurch gekennzeichnet, dass** die Modifikation im Bereich des Aktivierungspeptids eine Aktivierung des Faktor X-Analogons zu nativem Faktor Xa bzw, einem Faktor Xa-Analogon in vitro erlaubt.

13. Faktor X-Analogon nach Anspruch 12, **dadurch gekennzeichnet, dass** die Modifikation eine Aktivierung durch eine Protease, ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor IIa, Faktor XIIa, Faktor XIa, Faktor Xa, oder von Kallikrein, oder einem Derivat dieser Proteasen, erlaubt.

14. Faktor X-Analogon nach einem der Ansprüche 1 bis 11, **dadurch gekennzeichnet, dass** die Modifikation eine Aktivierung von Faktor X-Analogon zu nativem Faktor Xa bzw. einem Faktor Xa-Analogon in vivo erlaubt.

15. Faktor X-Analogon nach Anspruch 14, **dadurch gekennzeichnet, dass** die Modifikation eine Aktivierung durch eine Protease, ausgewählt aus der Gruppe der Serinproteasen, wie etwa Faktor XIIa, Faktor XIa, Faktor IIa, Faktor Xa oder durch Kallikrein erlaubt.

16. Faktor X-Analogon nach einem der Ansprüche 1 bis 15, **dadurch gekennzeichnet, dass** es als Faktor X-Analogon mit intaktem β-Peptid oder als ein C-terminal verkürztes Faktor-Analogon vorliegt.

17. Faktor X-Analogon nach einem der Ansprüche 1 bis 16, **dadurch gekennzeichnet, dass** es als einzelkettiges Molekül vorliegt.

18. Rekombinante DNA kodierend für ein Faktor X-Analogon nach einem der Ansprüche 1 bis 17, enthalten in einem Vektor zur rekombinanten Expression des kodierten Proteins,

19. Präparation enthaltend ein gereinigtes Faktor X-Analogon oder ein Vorläuferprotein davon mit einer Modifikation im Bereich der natürlicherweise vorkommenden Faktor X-Aktivierungsstelle mit einer Faktor X-Sequenz mit Gly228-R6-R5-R4-R3-R2-Arg234-R1, bezogen auf die Aminosäurenumerierung gemäß Fig . 1, wobei die Modifikation eine Prozessierungsstelle einer nicht-natürlicherweise in diesem Bereich der Faktor X-Sequenzspaltenden Protease darstellt und
a) R1 eine Aminosäure ausgewählt aus der Gruppe Ile, Val, Ser, Thr oder Ala
b) R2 eine Aminosäure ausgewählt aus der Gruppe Pro, Gly, Lys oder Arg
c) R3 eine Aminosäure ausgewählt aus der Gruppe Phe, Lys, Met, Gln, Glu, Ser, Val, Arg oder Pro
d) R4 eine Aminosäure ausgewählt aus der Gruppe Asp, Ile, Ser, Met, Pro, Thr, Arg oder Lys
e) R5 eine Aminosäure ausgewählt aus der Gruppe Asn, Lys, Ser, Glu, Ala, Gln, His oder Arg und
f) R6 eine Aminosäure ausgewählt aus der Gruppe Asp, Phe, Thr, Arg, Leu oder Ser ist.

20. Präparation nach Anspruch 19, **dadurch gekennzeichnet, dass** die Modifikation eine Spaltstelle für eine Protease, ausgewählt aus der Gruppe der bibasischen Endoproteasen, wie etwa Kexin/Kex2, Furin/PACE, PC1/PC3, PC2, PC4, PACE 4, LPC/PC7, der Serinproteasen, wie etwa Faktor IIa, Faktor XIIa, Faktor XIa, Faktor Xa, oder von Kallikrein darstellt.

21. Präparation nach einem der Ansprüche 19 oder 20, **dadurch gekennzeichnet, dass** das Faktor X-Analogon als Fxα-Analogon vorliegt.

22. Präparation nach einem der Ansprüche 19 oder 20, **dadurch gekennzeichnet, dass** das Faktor X-Analogon als C-terminal verkürztes Faktor X-Analogon vorliegt.

23. Präparation nach einem der Ansprüche 19 bis 22, **dadurch gekennzeichnet, dass** sie Faktor X-Analogon als einzelkettiges Molekül in isolierter Form enthält.

24. Präparation nach einem der Ansprüche 19 bis 23, **dadurch gekennzeichnet, dass** sie ein einzelkettiges Faktor X-Analogon in enzymatisch inaktiver Form mit einer Reinheit von mindestens 80%, vorzugsweise 90%, besonders bevorzugt von 95% und keine inaktiven, proteolytischen Intermediate von Faktor X/Xa-Analogon enthält.

25. Präparation nach einem der Ansprüche 19 bis 24, **dadurch gekennzeichnet, dass** sie Faktor X-Analogon als zweikettiges Molekül in isolierter Form enthält.

26. Präparation nach einem der Ansprüche 19 bis 25, **dadurch gekennzeichnet, dass** sie ein Faktor X-Analogon enthält, das eine Modifikation aufweist, die eine Aktivierung von Faktor X-Analogon zu nativem Faktor Xa bzw. einem Faktor Xa-Analogon in vitro erlaubt.

27. Präparation nach einem der Ansprüche 19 bis 26, **dadurch gekennzeichnet, dass** sie als pharmazeutisches Präparat formuliert ist.

28. Präparation nach einem der Ansprüche 19 bis 27, **dadurch gekennzeichnet, dass** sie in einer geeigneten Vorrichtung, vorzugsweise einer Applikationsvorrichtung, in Kombination mit einer Protease, ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor XIIa, Faktor XIa, Faktor Xa, Faktor IIa, oder von Kallikrein, oder einem Derivat dieser Proteasen, vorliegt.

29. Präparation nach Anspruch 28, **dadurch gekennzeichnet, dass** die Komponenten räumlich voneinander getrennt vorliegen.

30. Präparation nach einem der Ansprüche 19 bis 25, **dadurch gekennzeichnet, dass** sie ein Faktor X-Analogon enthält, das eine Modifikation aufweist, die eine Aktivierung von Faktor X-Analogon zu nativem Faktor Xa bzw. Faktor Xa-Analogon in vivo erlaubt .

31. Präparation enthaltend Faktor Xa-Analogon, die insbesondere frei ist von inaktiven Faktor X/Xa-Analogon-Intermediaten und autoproteolytischen Faktor X-Abbauprodukten, erhältlich durch Aktivierung eines Faktor X-Analogons gemäß einem der Ansprüche 1 bis 18.

32. Präparation nach einem der Ansprüche 19 bis 31, **dadurch gekennzeichnet, dass** sie einen physiologisch akzeptablen Träger enthält und in einer lagerstabilen Form vorliegt.

33. Präparation nach einem der Ansprüche 19 bis 32, **dadurch gekennzeichnet, dass** sie gegebenenfalls als weiteren Bestandteil einen Blutfaktor oder eine aktivierte Form eines Blutfaktors enthält.

34. Präparation nach Anspruch 33, **dadurch gekennzeichnet, dass** sie als weiteren Bestandteil mindestens eine Komponente mit Faktor VIII-Bypass-Aktivität enthält.

35. Präparation nach einem der Ansprüche 19 bis 34, **dadurch gekennzeichnet, dass** sie als pharmazeutische Zusammensetzung formuliert und gegebenenfalls als Mehrfachkomponentenpräparat vorliegt.

36. Verwendung einer Präparation nach einem der Ansprüche 19 bis 35 zur Herstellung eines Arzneimittels.

37. Verwendung einer Nukleinsäure nach Anspruch 18 zur Herstellung eines Arzneimittels.

38. Verfahren zur Herstellung einer Präparation enthaltend gereinigtes rekombinantes Faktor X-Analogon, **dadurch gekennzeichnet, dass** ein durch rekombinante Herstellung gewonnenes Faktor X-Analogon gemäß einem der Ansprüche 1 bis 17 isoliert und über ein chromatographisches Verfahren gereinigt wird.

39. Verfahren nach Anspruch 38, **dadurch gekennzeichnet, dass** es folgende Schritte umfasst:
- Bereitstellen einer Nukleinsäure gemäß Anspruch 18
- Transformation einer geeigneten Zelle
- Expression eines Faktor X-Analogons,
- gegebenenfalls Inkubieren des Faktor X-Analogons mit einer Protease
- Isolieren des Faktor X-Analogons und
- Reinigung des Faktor X-Analogons über ein chromatographisches verfahren.

40. Verfahren nach Anspruch 39, **dadurch gekennzeichnet, dass** das Faktor X-Analogon als zweikettiges Molekül isoliert wird.

41. Verfahren nach einem der Ansprüche 38 bis 40, **dadurch gekennzeichnet, dass** das zweikettige Faktor X-Analogon mit einer Protease, ausgewählt aus der Gruppe der Endoproteasen, wie etwa Kexin/Kex2, Furin/PACE, PC1/PC3, PC2, PC4, PACE 4, LPC/PC7, der Gruppe der Serinproteasen, wie etwa Faktor IIa, Faktor XIIa, Faktor XIa, Faktor Xa, oder von Kallikrein, oder einem Derivat dieser Proteasen, in Kontakt gebracht wird, unter Bedingungen unter denen das Faktor X-Analogon zu nativem Faktor Xa oder einem Faktor Xa-Analogon gespalten wird.

42. Verfahren nach Anspruch 41, **dadurch gekennzeichnet, dass** die Zelle eine Zelle ist, die eine Protease, die eine Spaltung der Einzelkette in leichte und schwere Kette von Faktor X bzw. eines Faktor X-Analogons ausführen kann, nicht exprimiert und gegebenenfalls eine Defizienz der Protease aufweist.

43. Verfahren nach Anspruch 42, **dadurch gekennzeichnet, dass** die Zelle keine Endoprotease, wie etwa Kexin, Furin, PACE oder ein Derivat davon exprimiert.

44. Verfahren nach einem der Ansprüche 42 oder 43, **dadurch gekennzeichnet, dass** das Faktor X-Analogon als einzelkettiges Molekül isoliert wird.

45. Verfahren nach Anspruch 44, **dadurch gekennzeichnet, dass** einzelkettiges, gegebenenfalls isoliertes Faktor X-Analogon mit einer Protease, ausgewählt aus der Gruppe der Endoproteasen, wie etwa Kexin/Kex2, Furin/PACE, PC1/PC3, PC2, PC4, PACE 4, LPC/PC7, oder einem Derivat dieser Proteasen in Kontakt gebracht wird, unter Bedingungen, unter denen einzelkettiges Faktor X-Analogon in die zweikettige Faktor X-Form gespalten wird.

46. Verfahren nach Anspruch 45, **dadurch gekennzeichnet, dass** einzelkettiges Faktor X-Analogon gegebenenfalls durch In-Kontakt-Bringen mit der Protease direkt zu Faktor Xa bzw. Faktor Xa-Analogon aktiviert wird.

47. Verfahren nach Anspruch 45, **dadurch gekennzeichnet, dass** zweikettiges Faktor X-Analogon mit einer weiteren, von der ersten verschiedenen Protease in Kontakt gebracht wird und zu Faktor Xa-Analogon oder nativem Faktor Xa aktiviert wird.

48. Verfahren nach einem der Ansprüche 38 bis 47, **dadurch gekennzeichnet, dass** die Protease immobilisiert ist.

49. Verfahren zur Herstellung einer Präparation enthaltend aktiven Faktor Xa bzw. Faktor Xa-Analogon, **dadurch gekennzeichnet, dass** ein gemäß einem Verfahren nach einem der Ansprüche 38 bis 45 hergestelltes Faktor X-Analogon einem Aktivierungsschritt unterzogen wird.

## Claims

1. Factor X analogue, **characterized in that** it has a modification in the region of the naturally occurring Factor Xa activation cleavage site with a Factor X sequence Gly228-R6-R5-R4-R3-R2-Arg234-R1, based on the amino acid numbering according to Fig. 1, wherein said modification represents a processing site for a protease not naturally cleaving in this region of the Factor X sequence, and
a) R1 is an amino acid selected from the group of Ile, Val, Ser, Thr or Ala,
b) R2 is an amino acid selected from the group of Pro, Gly, Lys or Arg,
c) R3 is an amino acid selected from the group of Phe, Lys, Met, Gln, Glu, Ser, Val, Arg or Pro,
d) R4 is an amino acid selected from the group of Asp, Ile, Ser, Met, Pro, Thr, Arg or Lys,
e) R5 is an amino acid selected from the group of Asn, Lys, Ser, Glu, Ala, Gln, His or Arg, and
f) R6 is an amino acid selected from the group of Asp, Phe, Thr, Arg, Leu or Ser.

2. Factor X analogue according to claim 1, **characterized in that** said modification concerns at least one amino acid within the amino acid sequence of the activation peptide.

3. Factor X analogue according to any one of claims 1 or 2, **characterized in that** said modification represents an exchange of at least one amino acid between Gly228 and Arg234, and optionally Ile235, based on the amino acid numbering according to Fig. 1.

4. Factor X analogue according to any one of claims 1 to 3, **characterized in that** said modification represents a processing site for a protease selected from the group of endoproteases, such as kexin/Kex2, furin/PACE, PC1/PC3, PC2, PC4, PACE 4, LPC/PC7, serine proteases, such as Factor IIa, Factor XIIa, Factor XIa, Factor Xa, or kallikrein, or a derivative of these proteases.

5. Factor X analogue according to any one of claims 1 to 4,
**characterized in that** it has a further modification in the region of the C-terminal Factor X amino acid sequence.

6. Factor X analogue according to claim 5, **characterized in that** it has a modification in the C-terminal region of the β-peptide cleavage site.

7. Factor X analogue according to claim 6, **characterized in that** said modification is a mutation, deletion or insertion in the region of the Factor X amino acid sequence between amino acid position Arg469 and Ser476.

8. Factor X analogue according to any one of claims 5 to 7, **characterized in that** said modification prevents the β-peptide from being cleaved off.

9. Factor X analogue according to claim 5, **characterized in that** it has a deletion of the Factor X β-peptide.

10. Factor X analogue according to claim 5, **characterized in that** it has a translation stop signal in the C-terminal region of the Factor X sequence.

11. Factor X analogue according to claim 10, **characterized in that** it has a translation stop signal at the position of amino acid 470 of the Factor X sequence.

12. Factor X analogue according to any one of claims 1 to 11, **characterized in that** said modification in the region of the activation peptide allows in vitro activation of the Factor X analogue to native Factor Xa or a Factor Xa analogue, respectively.

13. Factor X analogue according to claim 12, **characterized in that** said modification allows activation by a protease selected from the group of endoproteases, such as kexin/Kex2, furin/PACE, PC1/PC3, PC2, PC4, PACE 4, LPC/PC7, the group of serine proteases, such as Factor IIa, Factor XIIa, Factor XIa, Factor Xa, or kallikrein, or a derivative of these proteases.

14. Factor X analogue according to any one of claims 1 to 11, **characterized in that** said modification allows in vivo activation of Factor X analogue to native Factor Xa or a Factor Xa analogue, respectively.

15. Factor X analogue according to claim 14, **characterized in that** said modification allows activation by a protease selected from the group of serine proteases, such as Factor XIIa, Factor XIa, Factor IIa, Factor Xa, or kallikrein.

16. Factor X analogue according to any one of claims 1 to 15, **characterized in that** it is provided as a Factor X analogue having an intact β-peptide or as a Factor X analogue having a shortened C-terminus.

17. Factor X analogue according to any one of claims 1 to 16, **characterized in that** it is provided as a single chain molecule.

18. Recombinant DNA coding for a Factor X analogue according to any one of claims 1 to 17, contained in a vector for recombinant expression of the encoded protein.

19. Preparation containing a purified Factor X analogue or a precursor protein thereof having a modification in the region of the naturally occurring Factor X activation site having a Factor X sequence with Gly228-R6-R5-R4-R3-R2-Arg234-R1, based on the amino acid numbering according to Fig. 1, wherein said modification represents a processing site for a protease not naturally cleaving in this region of the Factor X sequence, and
a) R1 is an amino acid selected from the group of Ile, Val, Ser, Thr or Ala,
b) R2 is an amino acid selected from the group of Pro, Gly, Lys or Arg,
c) R3 is an amino acid selected from the group of Phe, Lys, Met, Gln, Glu, Ser, Val, Arg or Pro,
d) R4 is an amino acid selected from the group of Asp, Ile, Ser, Met, Pro, Thr, Arg or Lys,
e) R5 is an amino acid selected from the group of Asn, Lys, Ser, Glu, Ala, Gln, His or Arg, and
f) R6 is an amino acid selected from the group of Asp, Phe, Thr, Arg, Leu or Ser.

20. Preparation according to claim 19, **characterized in that** the modification represents a cleavage site for a protease selected from the group of dibasic endoproteases, such as kexin/Kex2, furin/PACE, PC1/PC3, PC2, PC4, PACE 4, LPC/PC7, serine proteases, such as Factor IIa, Factor XIIa, Factor XIa, Factor Xa, or kallikrein.

21. Preparation according to any one of claims 19 or 20, **characterized in that** the Factor X analogue is provided as Fxα analogue.

22. Preparation according to any one of claims 19 or 20, **characterized in that** the Factor X analogue is provided as a Factor X analogue having a shortened C-terminus.

23. Preparation according to any one of claims 19 to 22, **characterized in that** it contains Factor X analogue as a single chain molecule in isolated form.

24. Preparation according to any one of claims 19 to 23, **characterized in that** it contains a single chain Factor X analogue in enzymatically inactive form having a purity of at least 80%, preferably 90%, particularly preferably 95%, and that it does not contain inactive proteolytic intermediates of Factor X/Xa analogue.

25. Preparation according to any one of claims 19 to 24, **characterized in that** it contains Factor X analogue as a double chain molecule in isolated form.

26. Preparation according to any one of claims 19 to 25, **characterized in that** it contains Factor X analogue having a modification which allows in vitro activation of Factor X analogue to native Factor Xa or a Factor Xa analogue, respectively.

27. Preparation according to any one of claims 19 to 26, **characterized in that** it is formulated as a pharmaceutical preparation.

28. Preparation according to any one of claims 19 to 27, **characterized in that** it is provided in an appropriate device, preferably an application device, in combination with a protease selected from the group of endoproteases, such as kexin/Kex2, furin/PACE, PC1/PC3, PC2, PC4, PACE 4, LPC/PC7, of the group of serine proteases, such as Factor XIIa, Factor XIa, Factor Xa, Factor IIa or kallikrein, or a derivative of these proteases.

29. Preparation according to claim 28, **characterized in that** the components are provided spatially separated from each other.

30. Preparation according to any one of claims 19 to 25, **characterized in that** it contains a Factor X analogue having a modification allowing in vivo activation of Factor X analogue to native Factor Xa or Factor Xa analogue, respectively.

31. Preparation containing Factor Xa analogue, which is particularly free of inactive Factor X/Xa analogue intermediates and autoproteolytic Factor X degradation products, obtainable by activating a Factor X analogue according to any one of claims 1 to 18.

32. Preparation according to any one of claims 19 to 31, **characterized in that** it contains a physiologically acceptable carrier and is provided in stably storable form.

33. Preparation according to any one of claims 19 to 32, **characterized in that** it optionally contains a blood factor or an activated form of a blood factor as a further component.

34. Preparation according to claim 33, **characterized in that** it contains at least one component having Factor VIII bypass activity as a further component.

35. Preparation according to any one of claims 19 to 34, **characterized in that** it is formulated as a pharmaceutical composition and is optionally provided as a multiple component preparation.

36. Use of a preparation according to any one of claims 19 to 35 for the preparation of a medicament.

37. Use of a nucleic acid according to claim 18 for the preparation of a medicament.

38. Process for preparing a preparation containing purified recombinant Factor X analogue, **characterized in that** a Factor X analogue obtained by recombinant preparation is isolated according to any one of claims 1 to 17 and purified by means of a chromatographic process.

39. Process according to claim 38, **characterized in that** it comprises the following steps:
- providing a nucleic acid according to claim 18
- transformation of an appropriate cell
- expression of the Factor X analogue
- optional incubation of the Factor X analogue with a protease
- isolation of the Factor X analogue, and
- purification of the Factor X analogue by means of a chromatographic process.

40. Process according to claim 39, **characterized in that** said Factor X analogue is isolated as a double chain molecule.

41. Process according to any one of claims 38 to 40, **characterized in that** said double chain Factor X analogue is brought into contact with a protease selected from the group of endoproteases, such as kexin/Kex2, furin/PACE, PC1/PC3, PC2, PC4, PACE 4, LPC/PC7, the group of serine proteases, such as Factor IIa, Factor XIIa, Factor XIa, Factor Xa, or kallikrein, or a derivative of these proteases, under conditions under which said Factor X analogue is cleaved to native Factor Xa or a Factor Xa analogue.

42. Process according to claim 41, **characterized in that** the cell is a cell not expressing a protease that is capable of cleaving the single chain into light and heavy chain of Factor X or Factor X analogue, respectively, and which cell is optionally protease deficient.

43. Process according to claim 42, **characterized in that** said cell does not express an endoprotease, such as kexin, furin, PACE, or a derivative thereof.

44. Process according to any one of claims 42 or 43, **characterized in that** said Factor X analogue is isolated as a single chain molecule.

45. Process according to claim 44, **characterized in that** single chain, optionally isolated Factor X analogue is brought into contact with a protease selected from the group of endoproteases, such as kexin/Kex2, furin/PACE, PC1/PC3, PC2, PC4, PACE 4, LPC/PC7, or a derivative of these proteases, under conditions under which single chain Factor X analogue is cleaved to the double chain Factor X form.

46. Process according to claim 45, **characterized in that** a single chain Factor X analogue is activated directly to Factor Xa or Factor Xa analogue, respectively, optionally by bringing it into contact with the protease.

47. Process according to claim 45, **characterized in that** double chain Factor X analogue is brought into contact with another protease different from the first and is activated to Factor Xa analogue or native Factor Xa.

48. Process according to any one of claims 38 to 47, **characterized in that** said protease is immobilized.

49. Process of preparing a preparation containing active Factor Xa or Factor Xa analogue, respectively, **characterized in that** a Factor X analogue prepared according to a process according to any one of claims 38 to 45 is subjected to an activation step.

## Revendications

1. Analogue du facteur X, **caractérisé en ce qu'**il comprend une modification dans la zone du site de clivage naturel d'activation du facteur Xa, avec une séquence du facteur X Gly228-R6-R5-R4-R3-R2-Arg234-R1, la numérotation des acides aminés correspondant à la Figure 1, la modification représentant un site de maturation d'une protéase non naturelle, qui assure un clivage dans cette zone de la séquence du facteur X, et
a) R1 est un acide aminé choisi dans l'ensemble comprenant Ile, Val, Ser, Thr ou Ala,
b) R2 est un acide aminé choisi dans l'ensemble comprenant Pro, Gly, Lys ou Arg,
c) R3 est un acide aminé choisi dans l'ensemble comprenant Phe, Lys, Met, Gln, Glu, Ser, Val, Arg ou Pro,
d) R4 est un acide aminé choisi dans l'ensemble comprenant Asp, Ile, Ser, Met, Pro, Thr, Arg ou Lys,
e) R5 est un acide aminé choisi dans l'ensemble comprenant Asn, Lys, Ser, Glu, Ala, Gln, His ou Arg, et
f) R6 est un acide aminé choisi dans l'ensemble comprenant Asp, Phe, Thr, Arg, Leu ou Ser.

2. Analogue du facteur X selon la revendication 1, **caractérisé en ce que** la modification concerne au moins un acide aminé à l'intérieur de la séquence d'acides aminés du peptide d'activation.

3. Analogue du facteur X selon l'une des revendications 1 ou 2, **caractérisé en ce que** la modification représente un échange d'au moins l'un des acides aminés entre Gly228 et Arg234, et éventuellement Ile235, la numérotation des acides aminés correspondant à celle de la Figure 1.

4. Analogue du facteur X selon l'une des revendications 1 à 3, **caractérisé en ce que** la modification représente un site de maturation d'une protéase choisie dans l'ensemble des endoprotéases, notamment les protéases Kexine/Kex2, Furine/PACE, PC1/PC3, PC2, PC4, PACE 4, LPC/PC7, les protéases de la sérine, telles par exemple que le facteur IIa, le facteur XIIa, le facteur XIa, le facteur Xa, ou encore la kallikréine, ou un dérivé de ces protéases.

5. Analogue du facteur X selon l'une des revendications 1 à 4, **caractérisé en ce qu'**il comprend une modification supplémentaire dans la zone de la séquence d'acides aminés du facteur X C-terminale.

6. Analogue du facteur X selon la revendication 5, **caractérisé en ce qu'**il comprend une modification dans la zone C-terminale du site de clivage du β-peptide.

7. Analogue du facteur X selon la revendication 6, **caractérisé en ce que** la modification est une mutation, une délétion ou une insertion dans la zone de la séquence d'acides aminés du facteur X située entre les positions d'acides aminés Arg469 et Ser476.

8. Analogue du facteur X selon l'une des revendications 5 à 7, **caractérisé en ce que** la modification empêche un clivage du peptide β.

9. Analogue du facteur X selon la revendication 5, **caractérisé en ce qu'**il comprend une délétion du peptide β du facteur X.

10. Analogue du facteur X selon la revendication 5, **caractérisé en ce qu'**il comprend un signal d'arrêt de traduction dans la zone C-terminale de la séquence du facteur X.

11. Analogue du facteur X selon la revendication 10, **caractérisé en ce qu'**il comprend un signal d'arrêt de traduction sur la position de l'acide aminé 470 de la séquence du facteur X.

12. Analogue du facteur X selon l'une des revendications 1 à 11, **caractérisé en ce que** la modification autorise, dans la zone du peptide d'activation, une activation de l'analogue du facteur X en le facteur Xa natif ou en un analogue du facteur Xa in vitro.

13. Analogue du facteur X selon la revendication 12, **caractérisé en ce que** la modification autorise une activation par une protéase choisie dans l'ensemble comprenant les endoprotéases, telles par exemple que les protéases Kexine/Kex2, Furine/PACE, PC1/PC3, PC2, PC4, PACE 4, LPC/PC7, dans l'ensemble des protéases de la sérine, notamment le facteur IIa, le facteur XIIa, le facteur XIa, le facteur Xa, ou parmi la kallikréine, ou encore un dérivé de ces protéases.

14. Analogue du facteur X selon l'une des revendications 1 à 11, **caractérisé en ce que** la modification autorise une activation de l'analogue du facteur X en le facteur Xa natif ou en un analogue du facteur Xa in vivo.

15. Analogue du facteur X selon la revendication 14, **caractérisé en ce que** la modification autorise une activation par une protéase choisie parmi les protéases de la sérine telles par exemple que le facteur XIIa, le facteur XIa, le facteur IIa, le facteur Xa, ou par la kallikréine.

16. Analogue du facteur X selon l'une des revendications 1 à 15, **caractérisé en ce qu'**il se présente sous forme d'un analogue du facteur X, avec un peptide β intact, ou sous forme d'un analogue du facteur raccourci au niveau C-terminal.

17. Analogue du facteur X selon l'une des revendications 1 à 16, **caractérisé en ce qu'**il se présente sous forme d'une molécule monocaténaire.

18. ADN recombinant, codant pour un analogue du facteur X selon l'une des revendications 1 à 17, contenu dans un vecteur destiné à l'expression par recombinaison de la protéine codée.

19. Préparation contenant un analogue du facteur X purifié ou une protéine précurseur de ce dernier, avec une modification dans la zone du site d'activation naturel du facteur X avec une séquence du facteur X Gly228-R6-R5-R4-R3-R2-Arg234-R1, la numérotation des acides aminés correspondant à celle de la Figure 1, la modification représentant un site de maturation d'une protéase qui d'une manière non naturelle assure un clivage dans cette zone de la séquence du facteur X, et
a) R1 est un acide aminé choisi dans l'ensemble comprenant Ile, Val, Ser, Thr ou Ala,
b) R2 est un acide aminé choisi dans l'ensemble comprenant Pro, Gly, Lys ou Arg,
c) R3 est un acide aminé choisi dans l'ensemble comprenant Phe, Lys, Met, Gln, Glu, Ser, Val, Arg ou Pro,
d) R4 est un acide aminé choisi dans l'ensemble comprenant Asp, Ile, Ser, Met, Pro, Thr, Arg ou Lys,
e) R5 est un acide aminé choisi dans l'ensemble comprenant Asn, Lys, Ser, Glu, Ala, Gln, His ou Arg, et
f) R6 est un acide aminé choisi dans l'ensemble comprenant Asp, Phe, Thr, Arg, Leu ou Ser.

20. Préparation selon la revendication 19, **caractérisée en ce que** la modification représente un site de clivage pour une protéase choisie dans l'ensemble comprenant les endoprotéases bibasiques, telles par exemple que les protéases Kexine/Kex2, Furine/PACE, PC1/PC3, PC2, PC4, PACE 4, LPC/PC7, les protéases de la sérine, telles que le facteur IIa, le facteur XIIa, le facteur XIa, le facteur Xa, ou la kallikréine.

21. Préparation selon l'une des revendications 19 ou 20, **caractérisée en ce que** l'analogue du facteur X se présente sous forme d'un analogue Fxα.

22. Préparation selon l'une des revendications 19 ou 20, **caractérisée en ce que** l'analogue du facteur X se présente sous forme d'un analogue du facteur X raccourci au niveau C-terminal.

23. Préparation selon l'une des revendications 19 à 22, **caractérisée en ce qu'**elle contient l'analogue du facteur X en tant que molécule monocaténaire sous forme isolée.

24. Préparation selon l'une des revendications 19 à 23, **caractérisée en ce qu'**elle contient un analogue du facteur X monocaténaire sous une forme enzymatiquement inactivée, avec une pureté d'au moins 80 %, de préférence de 90 % et plus particulièrement de 95 %, et ne contient aucun intermédiaire protéolytique inactif de l'analogue du facteur X/Xa.

25. Préparation selon l'une des revendications 19 à 24, **caractérisée en ce qu'**elle contient l'analogue du facteur X sous forme d'une molécule bicaténaire sous forme isolée.

26. Préparation selon l'une des revendications 19 à 25, **caractérisée en ce qu'**elle contient un analogue du facteur X qui comporte une modification permettant une activation de l'analogue du facteur X en le facteur Xa natif ou un analogue du facteur Xa in vitro.

27. Préparation selon l'une des revendications 19 à 26, **caractérisée en ce qu'**elle est formulée sous forme d'une préparation pharmaceutique.

28. Préparation selon l'une des revendications 19 à 27, **caractérisée en ce qu'**elle se présente dans un dispositif approprié, de préférence dans un dispositif applicateur, en combinaison avec une protéase choisie dans l'ensemble comprenant les endoprotéases, telles par exemple que les protéases Kexine/Kex2, Furine/PACE, PC1/PC3, PC2, PC4, PACE 4, LPC/PC7, dans l'ensemble des protéases de la sérine, telles par exemple que le facteur XIIa, le facteur XIa, le facteur Xa, le facteur IIa, ou la kallikréine, ou un dérivé de ces protéases.

29. Préparation selon la revendication 28, **caractérisée en ce que** les composants se présentent spatialement séparés les uns des autres.

30. Préparation selon l'une des revendications 19 à 25, **caractérisée en ce qu'**elle contient un analogue du facteur X qui comprend une modification autorisant une activation de l'analogue du facteur X en le facteur Xa natif ou un analogue du facteur Xa.

31. Préparation contenant un analogue du facteur Xa, qui en particulier est exempte d'intermédiaires inactifs de l'analogue du facteur X/Xa et de produits de dégradation autoprotéolytique du facteur X, pouvant être obtenue par activation d'un analogue du facteur X selon l'une des revendications 1 à 18.

32. Préparation selon l'une des revendications 19 à 31, **caractérisée en ce qu'**elle contient un excipient acceptable d'un point de vue physiologique, et se présente sous une forme stable au stockage.

33. Préparation selon l'une des revendications 19 à 32, **caractérisée en ce qu'**elle contient éventuellement, en tant que constituant supplémentaire, un facteur sanguin ou une forme activée d'un facteur sanguin.

34. Préparation selon la revendication 33, **caractérisée en ce qu'**elle contient en tant que constituant supplémentaire au moins un composant ayant une activité de réduction de l'inhibition du facteur VIII.

35. Préparation selon l'une des revendications 19 à 34, **caractérisée en ce qu'**elle se présente sous forme d'une composition pharmaceutique et éventuellement sous forme d'une préparation à plusieurs composants.

36. Utilisation d'une préparation selon l'une des revendications 19 à 35 pour fabriquer un médicament.

37. Utilisation d'un acide nucléique selon la revendication 18 pour préparer un médicament.

38. Procédé de fabrication d'une préparation contenant un analogue recombinant purifié du facteur X, **caractérisé en ce qu'**on isole un analogue du facteur X, préparé par recombinaison, selon l'une des revendications 1 à 17, et on le purifie par un procédé chromatographique.

39. Procédé selon la revendication 38, **caractérisé en ce qu'**il comprend les étapes suivantes :
- mise à disposition d'un acide nucléique selon la revendication 18,
- transformation d'une cellule appropriée,
- expression d'un analogue du facteur X,
- éventuellement incubation de l'analogue du facteur X avec une protéase,
- isolement de l'analogue du facteur X et
- purification de l'analogue du facteur X par un procédé chromatographique.

40. Procédé selon la revendication 39, **caractérisé en ce que** l'analogue du facteur X est isolé sous forme d'une molécule bicaténaire.

41. Procédé selon l'une des revendications 38 à 40, **caractérisé en ce que** l'analogue du facteur X bicaténaire est mis en contact avec une protéase choisie dans l'ensemble des endoprotéases telles par exemple que les protéases Kexine/Kex2, Furine/PACE, PC1/PC3, PC2, PC4, PACE 4, LPC/PC7, le groupe des protéases de la sérine, notamment le facteur IIa, le facteur XIIa, le facteur XIa, le facteur Xa ou la kallikréine, ou un dérivé de ces protéases, dans des conditions dans lesquelles l'analogue du facteur X est clivé en le facteur Xa natif ou un analogue du facteur Xa.

42. Procédé selon la revendication 41, **caractérisé en ce que** la cellule est une cellule qui n'exprime pas une protéase pouvant exécuter un clivage d'une chaîne unique en une chaîne légère et une chaîne lourde du facteur X ou d'un analogue du facteur X, et éventuellement comprend une déficience de la protéase.

43. Procédé selon la revendication 42, **caractérisé en ce que** la cellule n'exprime aucune endoprotéase telle que la kexine, la furine, le PACE ou un dérivé de ces dernières.

44. Procédé selon l'une des revendications 42 ou 43, **caractérisé en ce que** l'analogue du facteur X est isolé sous forme d'une molécule monocaténaire.

45. Procédé selon la revendication 44, **caractérisé en ce qu'**on met en contact l'analogue du facteur X monocaténaire, éventuellement isolé, avec une protéine choisie dans l'ensemble comprenant les endoprotéases telles par exemple que les protéases Kexine/Kex2, Furine/PACE, PC1/PC3, PC2, PC4, PACE 4, LPC/PC7 ou un dérivé de ces protéases, dans des conditions dans lesquelles l'analogue monocaténaire du facteur X est clivé en la forme bicaténaire du facteur X.

46. Procédé selon la revendication 45, **caractérisé en ce que** l'analogue du facteur X monocaténaire est éventuellement, par mise en contact avec la protéase, directement activé en le facteur Xa ou l'analogue du facteur Xa.

47. Procédé selon la revendication 45, **caractérisé en ce que** l'analogue bicaténaire du facteur X est mis en contact avec une autre protéase, différente de la première, et est activé en l'analogue du facteur Xa ou en le facteur Xa natif.

48. Procédé selon l'une des revendications 38 à 47, **caractérisé en ce que** la protéase est immobilisée.

49. Procédé de fabrication d'une préparation contenant un facteur Xa actif ou un analogue actif du facteur Xa, **caractérisé en ce qu'**on soumet à une étape d'activation un analogue du facteur X préparé par un procédé selon l'une des revendications 38 à 45.


### SEQUENZPROTOKOLL

(1) ALLGEMEINE ANGABEN:
   (i) ANMELDER:
      (A) NAME: IMMUNO AG
      (B) STRASSE: Industriestrasse 67
      (C) ORT: Wien
      (D) BUNDESLAND: Austria
      (E) LAND: Austria
      (F) POSTLEITZAHL: 1220

      (A) NAME: Michele Himmelspach
      (B) STRASSE: Breitstetten 19
      (C) ORT: Leopoldsdorf
      (D) BUNDESLAND: Austria
      (E) LAND: Austria
      (F) POSTLEITZAHL: 2285

      (A) NAME: Uwe Schlokat
      (B) STRASSE: Hauptstrasse 51
      (C) ORT: Orth/Donau
      (D) BUNDESLAND: Austria
      (E) LAND: Austria
      (F) POSTLEITZAHL: 2304

      (A) NAME: Andreas Fisch
      (B) STRASSE: Wiener Strasse 14
      (C) ORT: Orth/Donau
      (D) BUNDESLAND: Austria
      (E) LAND: Austria
      (F) POSTLEITZAHL: 2304

      (A) NAME: Friedrich Dorner
      (B) STRASSE: Peterlinigasse 17
      (C) ORT: Wien
      (D) BUNDESLAND: Austria
      (E) LAND: Austria
      (F) POSTLEITZAHL: 1238

      (A) NAME: Johann Eibl
      (B) STRASSE: Gustav Tschermakgasse 2
      (C) ORT: Wien
      (D) BUNDESLAND: Austria
      (E) LAND: Austria
      (F) POSTLEITZAHL: 1180
   (ii) BEZEICHNUNG DER ERFINDUNG: Faktor X Analoge mit modifizierter Proteasespaltstelle
   (iii) ANZAHL DER SEQUENZEN: 27
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
   ATTACTCGAG AAGCTTACCA TGGGGCGCCC ACTG 34
(2) ANGABEN ZU SEQ ID NO: 2:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 2:
   ATTACAATTG CTGCAGGGAT CCAC 24
(2) ANGABEN ZU SEQ ID NO: 3:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
   CCCACAGGGC CCTACCCCTG T 21
(2) ANGABEN ZU SEQ ID NO: 4:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 4:
   ACCAGTTAAC CCTGGTGAAG TCGTTGTCGC CCCTCTC 37
(2) ANGABEN ZU SEQ ID NO: 5:
   (i) SEQUENZKENNZEICHEN:
      (A) LÄNGE: 28 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
   ACCATCGCGA CCTGGTCAGG TTGTTGTC 28
(2) ANGABEN ZU SEQ ID NO: 6:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 6:
   ACCAGAATCG ATTCTGGGTT TGATGTTGTC GCCCCTCTC 39
(2) ANGABEN ZU SEQ ID NO: 7:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 7:
   ACCAGAATCG ATTCTGGTCA TGCTGTTGTC GCCCCTCTC 39
(2) ANGABEN ZU SEQ ID NO: 8:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 8:
   ACCAGAATCG ATTCTTCCTT GGGGGTTGTC GCCCCTCTC 39
(2) ANGABEN ZU SEQ ID NO: 9:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 9:
   ACCAGAATCG ATTCTCGTTT TCATGTTGTC GCCCCTCTC 39
(2) ANGABEN ZU SEQ ID NO: 10:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 10:
   ACCAGAATCG ATTCTTCCCT CGATGTTGTC GCCCCTCTC 39
(2) ANGABEN ZU SEQ ID NO: 11:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 11:
   ACCAGAATCG ATTCTTTTCC TCCTGTTGTC GCCCCTCTC 39
(2) ANGABEN ZU SEQ ID NO: 12:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 12:
   ACCAGAATCG ATTCTCCTCA CCCTGTTGTC GCCCCTCTC 39
(2) ANGABEN ZU SEQ ID NO: 13:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 13:
   ACCAGAATCG ATTCTCCTCC TCCTGTTGTC GCCCCTCTC 39
(2) ANGABEN ZU SEQ ID NO: 14:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 14:
   ACCAGAATCG ATTCTTTTGG GCCTGTTGTC GCCCCTCTC 39
(2) ANGABEN ZU SEQ ID NO: 15:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 15:
   ACCAGAATCG ATTCTTTTCC TGATGTTGTC GCCCCTCTC 39
(2) ANGABEN ZU SEQ ID NO: 16:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 16:
   ACCAGAATCG ATTCTTTTGC TCCTGTTGTC GCCCCTCTC 39
(2) ANGABEN ZU SEQ ID NO: 17:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 17:
   ACCAGAATCG ATTCTGGTCA CCCTGTTGTC GCCCCTCTC 39
(2) ANGABEN ZU SEQ ID NO: 18:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 18:
   ACCAGAATCG ATTCTTTTGA GCCTGTTGTC GCCCCTCTC 39
(2) ANGABEN ZU SEQ ID NO: 19:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 19:
   ACCAGAATCG ATTCTCGTGC TCGTGTTGTC GCCCCTCTC 39
(2) ANGABEN ZU SEQ ID NO: 20:
   (i) SEQUENZKENNZEICHEN:
      (A) LÄNGE: 49 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 20:
   GTCACCGCCT TCCTCAAGTG GATCGACAGG TCCATGAAAA CCAGGTGAA 49
(2) ANGABEN ZU SEQ ID NO: 21:
   (i) SEQUENZKENNZEICHEN:
      (A) LÄNGE: 48 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 21:
   CTAGTTCACC TGGTTTTCAT GGACCTGTCG ATCCACTTGA GGAAGGCG 48
(2) ANGABEN ZU SEQ ID NO: 22:
   (i) SEQUENZKENNZEICHEN:
      (A) LÄNGE: 57 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 22:
   GTCACCGCCT TCCTCAAGTG GATCGACAGG TCCATGAAAA CCAAGGGCTT GCCCAAG 57
(2) ANGABEN ZU SEQ ID NO: 23:
   (i) SEQUENZKENNZEICHEN:
      (A) LÄNGE: 57 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 23:
   TTGGCCTTGG GCAAGCCCTT GGTTTTCATG GACCTGTCGA TCCACTTGAG GAAGGCG 57
(2) ANGABEN ZU SEQ ID NO: 24:
   (i) SEQUENZKENNZEICHEN:
      (A) LÄNGE: 55 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 24:
   GCCAAGAGCC ATGCCCCGGA GGTCATAACG TCCTCTCCAT TAAAGTGAGA TCCCA 55
(2) ANGABEN ZU SEQ ID NO: 25:
   (i) SEQUENZKENNZEICHEN:
      (A) LÄNGE: 54 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 25:
   CTAGTGGGAT CTCACTTTAA TGGAGAGGAC GTTATGACCT CCGGGGCATG GCTC 54
(2) ANGABEN ZU SEQ ID NO: 26:
   (i) SEQUENZKENNZEICHEN:
      (A) LÄNGE: 1467 Basenpaare
      (B) ART: Nucleotid
      (C) STRANGFORM: Einzelstrang
      (D) TOPOLOGIE: linear
      (ii) ART DES MOLEKÜLS: Genom-DNA
   (ix) MERKMAL:
      (A) NAME/SCHLÜSSEL: CDS
      (B) LAGE:1..1467
   (xi) SEQUENZBESCHREIBUNG: SEQ ID NO: 26:
   ATG GGG CGC CCA CTG CAC CTC GTC CTG CTC AGT GCC TCC CTG GCT GGC      48
   Met Gly Arg Pro Leu His Leu Val Leu Leu Ser Ala Ser Leu Ala Gly
    1               5                  10                  15
   CTC CTG CTG CTC GGG GAA AGT CTG TTC ATC CGC AGG GAG CAG GCC AAC      96
   Leu Leu Leu Leu Gly Glu Ser Leu Phe Ile Arg Arg Glu Gln Ala Asn
               20                  25                  30
   AAC ATC CTG GCG AGG GTC ACG AGG GCC AAT TCC TTT CTT GAA GAG ATG     144
   Asn Ile Leu Ala Arg Val Thr Arg Ala Asn Ser Phe Leu Glu Glu Met
           35                  40                  45
   AAG AAA GGA CAC CTC GAA AGA GAG TGC ATG GAA GAG ACC TGC TCA TAC     192
   Lys Lys Gly His Leu Glu Arg Glu Cys Met Glu Glu Thr Cys Ser Tyr
       50                  55                  60
   GAA GAG GCC CGC GAG GTC TTT GAG GAC AGC GAC AAG ACG AAT GAA TTC     240
   Glu Glu Ala Arg Glu Val Phe Glu Asp Ser Asp Lys Thr Asn Glu Phe
   65                  70                  75                  80
   TGG AAT AAA TAC AAA GAT GGC GAC CAG TGT GAG ACC AGT CCT TGC CAG     288
   Trp Asn Lys Tyr Lys Asp Gly Asp Gln Cys Glu Thr Ser Pro Cys Gln
                   85                  90                  95
   AAC CAG GGC AAA TGT AAA GAC GGC CTC GGG GAA TAC ACC TGC ACC TGT     336
   Asn Gln Gly Lys Cys Lys Asp Gly Leu Gly Glu Tyr Thr Cys Thr Cys
               100                 105                 110
   TTA GAA GGA TTC GAA GGC AAA AAC TGT GAA TTA TTC ACA CGG AAG CTC     384
   Leu Glu Gly Phe Glu Gly Lys Asn Cys Glu Leu Phe Thr Arg Lys Leu
           115                 120                 125
   TGC AGC CTG GAC AAC GGG GAC TGT GAC CAG TTC TGC CAC GAG GAA CAG     432
   Cys Ser Leu Asp Asn Gly Asp Cys Asp Gln Phe Cys His Glu Glu Gln
       130                 135                 140
   AAC TCT GTG GTG TGC TCC TGC GCC CGC GGG TAC ACC CTG GCT GAC AAC     480
   Asn Ser Val Val Cys Ser Cys Ala Arg Gly Tyr Thr Leu Ala Asp Asn
   145                 150                 155                 160
   GGC AAG GCC TGC ATT CCC ACA GGG CCC TAC CCC TGT GGG AAA CAG ACC     528
   Gly Lys Ala Cys Ile Pro Thr Gly Pro Tyr Pro Cys Gly Lys Gln Thr
                   165                 170                 175
   CTG GAA CGC AGG AAG AGG TCA GTG GCC CAG GCC ACC AGC AGC AGC GGG     576
   Leu Glu Arg Arg Lys Arg Ser Val Ala Gln Ala Thr Ser Ser Ser Gly
               180                 185                 190
   GAG GCC CCT GAC AGC ATC ACA TGG AAG CCA TAT GAT GCA GCC GAC CTG     624
   Glu Ala Pro Asp Ser Ile Thr Trp Lys Pro Tyr Asp Ala Ala Asp Leu
           195                 200                 205
   GAC CCC ACC GAG AAC CCC TTC GAC CTG CTT GAC TTC AAC CAG ACG CAG      672
   Asp Pro Thr Glu Asn Pro Phe Asp Leu Leu Asp Phe Asn Gln Thr Gln
       210                 215                 220
   CCT GAG AGG GGC GAC AAC AAC CTC ACC AGG ATC GTG GGA GGC CAG GAA      720
   Pro Glu Arg Gly Asp Asn Asn Leu Thr Arg Ile Val Gly Gly Gln Glu
   225                 230                 235                 240
   TGC AAG GAC GGG GAG TGT CCC TGG CAG GCC CTG CTC ATC AAT GAG GAA      768
   Cys Lys Asp Gly Glu Cys Pro Trp Gln Ala Leu Leu Ile Asn Glu Glu
                   245                 250                 255
   AAC GAG GGT TTC TGT GGT GGA ACT ATT CTG AGC GAG TTC TAC ATC CTA      816
   Asn Glu Gly Phe Cys Gly Gly Thr Ile Leu Ser Glu Phe Tyr Ile Leu
               260                 265                 270
   ACG GCA GCC CAC TGT CTC TAC CAA GCC AAG AGA TTC AAG GTG AGG GTA      864
   Thr Ala Ala His Cys Leu Tyr Gln Ala Lys Arg Phe Lys Val Arg Val
           275                 280                 285
   GGG GAC CGG AAC ACG GAG CAG GAG GAG GGC GGT GAG GCG GTG CAC GAG      912
   Gly Asp Arg Asn Thr Glu Gln Glu Glu Gly Gly Glu Ala Val His Glu
       290                 295                 300
   GTG GAG GTG GTC ATC AAG CAC AAC CGG TTC ACA AAG GAG ACC TAT GAC      960
   Val Glu Val Val Ile Lys His Asn Arg Phe Thr Lys Glu Thr Tyr Asp
   305                 310                 315                 320
   TTC GAC ATC GCC GTG CTC CGG CTC AAG ACC CCC ATC ACC TTC CGC ATG     1008
   Phe Asp Ile Ala Val Leu Arg Leu Lys Thr Pro Ile Thr Phe Arg Met
                   325                 330                 335
   AAC GTG GCG CCT GCC TGC CTC CCC GAG CGT GAC TGG GCC GAG TCC ACG     1056
   Asn Val Ala Pro Ala Cys Leu Pro Glu Arg Asp Trp Ala Glu Ser Thr
               340                 345                 350
   CTG ATG ACG CAG AAG ACG GGG ATT GTG AGC GGC TTC GGG CGC ACC CAC     1104
   Leu Met Thr Gln Lys Thr Gly Ile Val Ser Gly Phe Gly Arg Thr His
           355                 360                 365
   GAG AAG GGC CGG CAG TCC ACC AGG CTC AAG ATG CTG GAG GTG CCC TAC     1152
   Glu Lys Gly Arg Gln Ser Thr Arg Leu Lys Met Leu Glu Val Pro Tyr
       370                 375                 380
   GTG GAC CGC AAC AGC TGC AAG CTG TCC AGC AGC TTC ATC ATC ACC CAG     1200
   Val Asp Arg Asn Ser Cys Lys Leu Ser Ser Ser Phe Ile Ile Thr Gln
   385                 390                 395                 400
   AAC ATG TTC TGT GCC GGC TAC GAC ACC AAG CAG GAG GAT GCC TGC CAG     1248
   Asn Met Phe Cys Ala Gly Tyr Asp Thr Lys Gln Glu Asp Ala Cys Gln
                   405                 410                 415
   GGG GAC AGC GGG GGC CCG CAC GTC ACC CGC TTC AAG GAC ACC TAC TTC     1296
   Gly Asp Ser Gly Gly Pro His Val Thr Arg Phe Lys Asp Thr Tyr Phe
               420                 425                 430
   GTG ACA GGC ATC GTC AGC TGG GGA GAG AGC TGT GCC CGT AAG GGG AAG     1344
   Val Thr Gly Ile Val Ser Trp Gly Glu Ser Cys Ala Arg Lys Gly Lys
           435                 440                 445
   TAC GGG ATC TAC ACC AAG GTC ACC GCC TTC CTC AAG TGG ATC GAC AGG    1392
   Tyr Gly Ile Tyr Thr Lys Val Thr Ala Phe Leu Lys Trp Ile Asp Arg
       450                 455                 460
   TCC ATG AAA ACC AGG GGC TTG CCC AAG GCC AAG AGC CAT GCC CCG GAG    1440
   Ser Met Lys Thr Arg Gly Leu Pro Lys Ala Lys Ser His Ala Pro Glu
   465                 470                 475                 480
   GTC ATA ACG TCC TCT CCA TTA AAG TGA                                1467
   Val Ile Thr Ser Ser Pro Leu Lys  *
                   485
(2) ANGABEN ZU SEQ ID NO: 27:
   (i) SEQUENZKENNZEICHEN:
      (A) L NGE: 489 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 27: